# EUROPÄISCHE PATENTSCHRIFT

(11) **EP 2 650 347 B1**
(45) Veröffentlichungstag und Bekanntmachung des Hinweises auf die Patenterteilung: **01.11.2017**
(21) Anmeldenummer: 13150456.5
(22) Anmeldetag: 08.01.2013
(51) Int. Cl.: C10K 1/08, C10K 1/02, C10K 1/16, C12M 1/00, C10K 1/10, C10B 49/02, C10B 53/00, C12P 5/02

(54) **Kombi-Mischgasanlage mit Gasreinigung**
Combined mixed gas system with gas purification
Poste de mélange combiné avec nettoyage de gaz et installation de nettoyage de gaz

(30) Priorität: 31.01.2012 DE 102012001728; 20.02.2012 DE 102012003246; 22.02.2012 DE 102012003463
(43) Veröffentlichungstag der Anmeldung: 16.10.2013
(73) Patentinhaber: extech bioenergy GmbH & Co.KG, 86947 Geretshausen-Weil (DE)
(72) Erfinder: Gaugenrieder, Fritz, 86947 Geratshausen (DE); Schiffner, Eberhard, 98631 Grabfeld (DE)
(74) Vertreter: Mollekopf, Gerd Willi

(56) Entgegenhaltungen:
- WO-A2-2007/048058
- DE-A1-102010 012 487
- US-A1- 2006 260 190
- US-A1- 2010 298 450
- US-A1- 2011 138 684

## Beschreibung

Die Erfindung betrifft eine Kombi-Mischgasanlage mit einer Verschwelungseinheit zur Erzeugung von Verschwelungsgasen, einer Verschwelungsgasreinigungseinheit und optional einem Fermenter zur Umsetzung des gereinigten Verschwelungsgases in Mischgas (Biogas). Weiterhin betrifft die Erfindung eine Gasreinigungseinrichtung zur Reinigung von Gasen oder Verschwelungsgasen, insbesondere zum Ausfiltern von Teerbestandteilen aus einem Gasstrom.

Aus der EP 1 118 671 A1 ist eine Kombi-Biogasanlage bekannt, bei der organische Stoffe zunächst einem Fermentationsreaktor zur aeroben Umsetzung zugeführt werden. Die Abgase und feste bzw. flüssige Rückstände aus dem Fermentationsreaktor werden einem Holzvergaser zugeführt, wo die Stoffe thermisch umgesetzt und zu Holzgas und Holzkohlenprodukte umgewandelt werden. Das Holzgas wird optional durch einen Gasreiniger geleitet und dann einer methanogenesen Fermentation zugeführt. Bei der methanogenesen Fermentation wird das Holzgas optional unter Zusetzung weiterer Gase in Biogas umgewandelt. Als Endprodukte aus der Umsetzung des organischen Stoffes stehen damit einerseits Biogas und andererseits Holzkohleprodukte zur Verfügung.

Aus der DE 10 2004 024 672 B4 ist ein Mehrzonen-Vergaser zur Erzeugung von teerfreiem Verschwelungsgas bekannt, in dem Biomasse vergast werden kann. Das zu verschwelende Material wird einer Pyrolyse- und Trocknungszone zugeführt, von wo es über einen Pendelrost in eine zweistufige Crackzone gelangt, deren Zonen durch einen weiteren Pendelrost getrennt sind. Von der Crackzone gelangt das Material in eine Nachvergasungseinheit, der Sauerstoff für einen Oxidations- und Reduktionsprozess zugeführt wird und aus der als Abfallprodukt Asche entnommen wird. Das teerhaltige Verschwelungsgas aus der Nachvergasungseinheit wird unter geregelter Zufuhr von Sauerstoff teilweise verbrannt, wodurch ein Teil des Teers gecrackt wird. Die teilverbrannten Verschwelungsgase aus der Nachvergasungseinheit werden gemischt mit dem Verschwelungsgas der ersten Crackstufe in die Materialbetten der ersten und zweiten Crackstufe zugeführt. Das in der zweiten Crackstufe entnommene teerarme Verschwelungsgas wird als Biogas entnommen.

Beim Holzvergaser nach der DE 20 2005 019 717 U1 werden Hackschnitzel von unten in einen Mehrzonenvergaser eingeführt. Die Zonen des Vergasers gehen kontinuierlich ineinander über, wobei auf eine Pyrolysezone eine Oxidationszone und auf diese eine Reduktionszone folgt. Im Zufuhrbereich für die Hackschnitzel wird den Hackschnitzeln zum Trocknen Abgas aus einem Blockheizkraftwerk zugeführt. Das Verschwelungsgas aus dem Holzvergaser wird über einen Gasreiniger dem BHKW zugeführt.

Die folgenden Schriften betreffen eine Verschwelungseinrichtung: DE 10 2004 024 672 B4, DE 20 2005 019 717 U1, DE 10 2009 031 596 A1, DE 10 2008 014 799 A1, DE 601 20 957 T2, und DE 10 2010 012 487 A1. In Hinsicht auf die Gasreinigung betrifft oder erwähnt:
- DE 10 2004 024 672 B4 die Staubreinigung vor der Verbrennung des Synthesegases [S. 9, links, Z. 24-35]
- DE 10 2009 031 596 A1 die Reinigung des Synthesegases [0047],
- DE 20 2005 019 717 U1 eine Gasreinigung [0022],
- DE 10 2008 014 799 A1 eine Synthesegastrennung und teilweise Rückführung in den Pyrolyseprozess [0024],
- DE 601 20 957 T2 ein Reinigungsgerät zur Gasreinigung [0043].
- DE 10 2010 012 487 A1 einen Gaswäscher unter Verwendung von RME [0053],

Die US 2006/0260190 A1 beschreibt eine Kombi-Mischgaseinrichtung mit einer Verschwelungseinrichtung, in der biologisches Rohmaterial verschwelt wird. Das Verschwelungsgas wird durch einen Wärmetauscher abgekühlt und dann einem weiteren Gaskühlungsprozess und einem Gasreinigungsprozess zugeführt. Ein Teil eines zugeführten, behandelten Abwassers wird als Reinigungsmedium dem Gasreinigungsprozess zugeführt. Beim Reinigungsprozess wird dem Gas Staub entzogen, bevor es einer Gasturbine für einen Energiegewinnungsprozess zur Verfügung gestellt wird. Abwasser aus dem Gasreinigungsprozess wird als Abwasser zu einer Abwasserbehandlungseinheit abgeführt. Der abgeschiedene Staub aus dem Gasreinigungsprozess wird in die Verschwelungseinrichtung zurückgeführt.

US 2010/0298450 A1 offenbart eine Vorrichtung zur Verschwelung von Biomasse und zur Erzeugung von Wasserstoff und Methangas. In einer Verschwelungseinrichtung werden Verschwelungsgase erzeugt, die durch ein Abscheidesystem getrennt werden von Verkohlungsrückständen. Die Verkohlungsrückstände werden einem Verbrennungsreaktor zugeführt, dessen Abgase in einem Separator getrennt werden in Verbrennungsgase und ein heißes Medium). Das heiße Medium fällt in einen Konditionierungsreaktor, in den die Verschwelungsgase der Verschwelungseinrichtung zugeführt werden. Das heiße Medium bindet Teerbestandteile, die dann zusammen mit dem heißen Medium in die Verschwelungseinrichtung zurückgeführt werden.

Die Erfindung ist im Anspruch 1 bzw. 11 definiert. Vorteilhafte Ausgestaltungen sind Gegenstand von Unteransprüchen.

Hinsichtlich der Herstellung von möglichst methanreichem Biogas ist es bei der o.g. EP 1 118 671 A1 unter praktischen Gesichtspunkten nachteilig, dass rein organische Stoffe als Ausgangsstoffe beim aeroben Fermentationsreaktor zur Verfügung stehen müssen. Andererseits ist es nachteilig, dass Holzkohleprodukte zur Verfügung gestellt werden, die wiederum einem separaten Verarbeitungs-, Vertriebs- und Verwendungslcreislauf zugeführt werden müssen. Es ist auch zu erwarten, dass die Holzkohleprodukte aus der Verschwelung teerbelastet sind und somit die Holzkohleprodukte bei der Weiterverwertung hohen Umweltauflagen unterliegen.

Gemäß Anspruch 1 ist eine Kombi-Mischgasanlage zur Erzeugung von methanhaltigem Gas vorgesehen. Die Kombianlage umfasst eine Verschwelungseinrichtung (auch Pyrolysator oder 'Holzvergaser'), in der durch thermische Prozesse Verschwelungsgase erzeugt werden. Als Ausgangsmaterial, das in der Verschwelungseinrichtung verschwelt wird, wird kohlenstoffhaltiges Material verwendet, was sowohl organisches Material als auch nicht-organisches Material einschließen kann. Sowohl das biologische Material wie auch das nicht-biologische Material können Abfälle und/oder Rohstoffe/-produkte sein. Die Verschwelungsgase von der Verschwelungseinrichtung werden einer nachgeschalteten Gasreinigungseinrichtung zugeführt, um die Verschwelungsgase in der Gasreinigungseinrichtung zu reinigen. Im Verschwelungsgasstrom von der Verschwelungseinrichtung zur Gasreinigungseinrichtung werden als gasförmige und/oder dampfförmige und/oder staub- bzw. feststoffförmige Verschwelungsprodukte ein oder mehrere der folgenden Verunreinigungen mitgeführt: Wasserdampf, Asche, Teer in Partikelform (als Kondensat) oder dampfförmig oder dergleichen. Die Asche oder Feststoffpartikel können organischer oder anorganischer Natur sein (beispielsweise mineralische oder kohlenstoffhaltige Asche oder Feststoffpartikel).

Mittels der Gasreinigungseinrichtung werden die Verunreinigungen vollständig oder weitgehend aus dem Verschwelungsgasstrom entfernt. Verschwelungsgas umfasst hier hauptsächlich H₂, Methan, CO und CO₂, kann aber auch andere Verschwelungsgase enthalten.

Optional werden die gereinigten Verschwelungsgase aus der Gasreinigungseinrichtung einer nachgeschalteten Fermentationseinrichtung zugeführt, um diese in ein methan- und/oder Wasserstoff-angereichertes Gas ('Biogas') umzuwandeln. Vorzugsweise wird die Fermentationseinrichtung neben dem gereinigten Verschwelungsgas mit kohlenstoffhaltigem Material beschickt (oder anderen üblichen Einsatzstoffen), das dort durch einen Fermentationsprozess unterstützt mit den gereinigten Verschwelungsgasen zu dem Methan-angereicherten Gas umgesetzt wird. Vorzugsweise ist das der Fermentationseinrichtung zugeführte Material ausschließlich organisches Material (biologische Abfälle wie land-/forstwirtschaftliche Erzeugnisse). Vorzugsweise wird die Zusammensetzung der Verschwelungsgase in der Fermentation durch die bakterielle Umsetzung so geändert, dass sich der Anteil von CH₄ zu Lasten des Anteils an H₂ und CO erhöht. Die Kombi-Mischgasanlage kann auch ohne die Fermentationseinrichtung zum Einsatz kommen.

Erfindungsgemäß werden in der Gasreinigungseinrichtung Teerbestandteile (in der Dampfphase und/oder in der Feststoffphase (Teerpartikel)) aus den Verschwelungsgasen entfernt, so dass die gereinigten Verschwelungsgase frei oder weitgehend frei von Teerrückständen sind. Der optionale Fermentationsprozess in der optionalen Fermentationseinrichtung reagiert empfindlich auf die Zufuhr von Teer, wobei der Fermentationsprozess bei zu hohem Teeranteil oder zu hoher Teerzufuhr entweder nicht optimal verläuft oder sogar die Bakterienflora an sich abgetötet wird. Daher ist es wichtig, den Teer bzw. die Teerbestandteile aus den Verschwelungsgasen zu entfernen. Weiterhin soll das Material, das aus der Fermentationseinrichtung als Gärrest (eines der angestrebten Endprodukte) entfernt wird, teerfrei sein, so dass es ohne weitere Behandlung direkt weiter verwertet werden kann, beispielsweise als Dünger in der Landwirtschaft. Dies wird im ausreichenden Maße durch die Entfernung bzw. signifikante Reduzierung des Teeranteils bei der Reinigung in der Gasreinigungseinrichtung erreicht. Vorzugsweise werden die Teerbestandteile durch Umwandlung entfernt. Beispielsweise werden die Teerbestandteile in der Gasreinigungseinrichtung mit einem Lösungsmittel aufgelöst und in eine Lösungsphase überführt, bei der die Kohlenstoffketten des Teers zumindest teilweise zerlegt sind. Vorzugsweise erfolgt die Umwandlung durch Umesterung und/oder Veresterung.

Zusätzlich werden die in der Gasreinigungseinrichtung herausgefilterten bzw. zurückgehaltenen Teerbestandteile durch eine Rückfuhrleitung zwischen der Gasreinigungseinrichtung und der Verschwelungseinrichtung in die Verschwelungseinrichtung zurückgeführt. Die Teerbestandteile sind kohlenstofflialtig und können daher ebenfalls als Ausgangsmaterial zur Erzeugung von Methan und CO₂ sowie anderen kohlenstoffhaltigen Gasen in der Verschwelungseinrichtung umgesetzt werden. Es ist daher durch die Rückführung der Teerbestandteile in den Verschwelungsprozess nicht notwendig, den Teer als Abfallmaterial einer Sondermüllbehandlung zuzuführen, sondern es wird so lange im Kreislauf zwischen Verschwelungseinrichtung und Gasreinigungseinrichtung durchlaufen lassen, bis eine im Wesentlichen vollständige oder vollständige Umsetzung in zur Mischgaserzeugung verwertbare Verschwelungsgaskomponenten stattgefunden hat.

Die Reinigungseinrichtung weist einen Gaswäscher auf, in dem Mittels eines Reinigungsmediums nicht-gasförmige Bestandteile aus dem Verschwelungsgasstrom ausgeschieden und/oder umgesetzt werden.

Die Reinigungseinrichtung weist einen Gaswäscher und eine getrennt vom Gaswäscher angeordnete Trenneinheit auf.

In der Trenneinheit werden das Medium und die herausgefilterten bzw. umgesetzten Verunreinigungsbestandteile aus dem Verschwelungsgasstrom in verschiedene Phasen bzw. Phasentypen aufgetrennt. Beispielsweise ist die Trenneinheit ein Mediumbehälter, in dem sich verschiedene Schichtungen der verschiedenen Phasen bzw. Phasentypen ausbilden.

Bei der erfindungsgemäßen Kombi-Mischgasanlage wird somit das ursprünglich der Verschwelungseinrichtung zugeführte kohlenstoffhaltige Material betreffend den Kohlenstoffanteil möglichst weitgehend in Verschwelungsgas umgesetzt, wobei die prozessbedingt Bestandteile (Störstoffe) entstehen, die in der Kombi-Mischgasanlage soweit behandelt werden, dass eine Weiterbehandlung oder Entsorgung in betriebsfremden Verwertungswegen unnötig wird. D.h. es müssen keine getrennten Verwertungswege für die Endprodukte der Verschwelung und Gasreinigung z.B. hinsichtlich der Kohlenstoffprodukte (z.B. Holzkohle und Teer) vorgesehen werden.

Gemäß einer vorteilhaften Ausgestaltung ist die Gasreinigungseinrichtung weiterhin dazu ausgelegt, eine oder alle der folgenden Verunreinigungsbestandteile aus dem Verschwelungsgasstrom zu entfernen oder herauszufiltern: Lösungsmittel-lösliche Stoffe (z.B. Teer wie oben beschrieben); Feststoffbestandteile (beispielsweise Asche), die im Verschwelungsgasstrom von der Verschwelungseinrichtung mitgeführt werden; wasserlösliche Stoffe, die ebenfalls im Verschwelungsgasstrom mitgeführt werden, wie beispielsweise wasserlösliche Gase, wasserlösliche Feststoffbestandteile oder Flüssigkeitströpfchen oder dampfförmige Phasen aus der Zersetzung in der Verschwelungseinrichtung; Wasserdampf der vorzugsweise aufgrund der Abkühlung des Verschwelungsgasstroms in der Gasreinigungseinrichtung auskondensiert.

Vorzugsweise ist die Gasreinigungseinrichtung dazu ausgelegt, die Temperatur des Verschwelungsgasstromes herunter zu kühlen, so dass die kondensierbaren Gase oder die Dämpfe in der Gasreinigungseinrichtung kondensieren und so aus dem Verschwelungsgasstrom entfernt werden. Vorzugsweise wird die aus der Abkühlung des Verschwelungsgasstroms in der Gasreinigungseinrichtung freigesetzte Wärme als Prozesswärme genutzt und innerhalb der Kombi-Mischgasanlage weiterverwendet. Beispielsweise wird die in der Gasreinigungseinrichtung gewonnene Prozesswärme dazu verwendet, das kohlenstoffhaltige Ausgangsmaterial, das der Verschwelungseinrichtung zugeführt wird oder ein biologisches Ausgangsmaterial, das der Fermentationseinrichtung zur Umsetzung in Mischgas zugeführt wird, vorzuwärmen und/oder zu trocknen.

Gemäß einer vorteilhaften Ausgestaltung werden in der Gasreinigungseinrichtung leicht kondensierbare Gase und/oder dampfförmige Stoffe durch Abkühlung kondensiert (siehe oben) und das Kondensat wird gleichzeitig als ausschließliches Reinigungsmedium in der Gasreinigungseinrichtung verwendet oder als Bestandteil eines Gemisches von Reinigungsmedien oder verschiedener Phasen von Reinigungsmedien in der Gasreinigungseinrichtung verwendet. Vorzugsweise ist die durch Abkühlung kondensierte Flüssigkeit ausschließlich Wasser oder überwiegend Wasser, welches dann zur Lösung von wasserlöslichen Bestandteilen aus dem Verschwelungsgasstrom verwendet wird. Beispielsweise lassen sich Salze bzw. mineralische Bestandteile im Wasser lösen, so dass diese aus dem Verschwelungsgasstrom in Lösung im Wasser übergehen.

Besonders vorteilhaft werden die aus dem Verschwelungsgasstrom zurück gehaltenen oder herausgefilterten Bestandteile in verschiedene Phasen aufgetrennt (beispielsweise Lösungen wie wässrige Lösungen oder Lösungen in organischen oder anorganischen Lösungsmitteln) und/oder als Feststoffbestandteile beispielsweise mittels schwerkraftbedingter Absetzung und/oder Zyklonabscheidung oder dergleichen von den anderen Phasen getrennt. Besonders bevorzugt wird als gereinigtes Verschwelungsgas aus der Gasreinigungseinrichtung nur noch die Gasphase derjenigen Bestandteile in die Fermentationseinrichtung überführt, die in der Gasreinigungseinrichtung weder umgesetzt, kondensiert und/oder als Feststoffe abgesetzt wurden. Das Reinigungsmedium im Gaswäscher ist eine Flüssigkeit oder ein Flüssigkeitsgemisch oder der Gaswäscher arbeitet in mehreren Stufen mit jeweils unterschiedlichen Medien, vorzugsweise mit unterschiedlichen Flüssigkeiten. Ganz besonders vorteilhaft arbeitet der Gaswäscher mit Wasser als einem Lösungsmittel zum Auflösen von wasserlöslichen Bestandteilen aus dem Verschwelungsgasstrom und/oder mit einem organischen und/oder anorganischen Lösungsmittel zum Auflösen von organischen bzw. anorganischen Bestandteilen. Wie erwähnt, kann der Gaswäscher mehrstufig sein, so dass der Verschwelungsgasstrom zuerst in der einen Flüssigkeit (z.B. Wasser) und dann in der anderen Flüssigkeit (z.B. organisches/anorganisches Lösungsmittel) oder umgekehrt gereinigt wird. Vorzugsweise liegt das Reinigungsmedium aber als Gemisch vor, wobei dieses vorzugsweise mechanisch, hydraulisch und/oder durch die Strömung des Verschwelungsgasstroms durchmischt wird, so dass der Verschwelungsgasstrom mit den zumindest zwei Flüssigkeitsbestandteilen in Kontakt kommt.

Ganz besonders vorteilhaft muss bei Verwendung von Wasser als Reinigungsmedium (ausschließliches Medium oder in getrennten Stufen oder im Gemisch vorliegend) das Wasser nicht separat von Außen dem Reinigungsprozess zugeführt werden, sondern wird wie oben beschrieben durch Kondensation des im Verschwelungsgasstrom mitgeführten Wasserdampfes gewonnen.

Gemäß einer Ausführungsform sind der Gaswäscher und die Trenneinheit über zumindest eine Fluidleitung miteinander verbunden und ist das Medium zum Reinigen des Verschwelungsgasstroms im Gaswäscher eine Flüssigkeit oder ein Flüssigkeitsgemisch (siehe oben). Diese Aufteilung hat den Vorteil, dass im Gaswäscher das Medium hydraulisch oder mechanisch betätigt mit dem Verschwelungsgasstrom durchmischt werden kann, während das mit den vom Verschwelungsgasstrom zu trennenden Bestandteilen angereicherte Medium in die Trenneinheit überführt wird.

Vorzugsweise wird in der Trenneinheit das Reinigungsmedium, das im Gaswäscher benötigt wird, oder ein Bestandteil des Reinigungsmediums, durch die Phasentrennung angereichert und zur Reinigung in den Gaswäscher zurückgeführt. Beispielsweise kann die Trenneinheit eine Auftrennung in eine flüssige Phase mit geringem Feststoffanteil und in eine flüssige Phase mit hohem Feststoffanteil herbeiführen, vorzugsweise durch schwerkraftbedingte Absetzung der Feststoffbestandteile. Weiterhin kann die flüssige Phase sich in verschiedene, nicht-mischbare Flüssigkeitsbestandteile separieren, wie beispielsweise eine oder mehrere der folgenden Flüssigkeiten: organisches Lösungsmittel, anorganisches Lösungsmittel, Öl und Wasser.

Besonders vorteilhaft ist die Trenneinheit mittels einer Zirkulationseinheit mit dem Gaswäscher so verbunden, dass zwischen diesen ein Medium-Fluidkreislauf herstellbar ist. Beispielsweise sind der Gaswäscher und die Trenneinheit mit zumindest zwei Fluidleitungen verbunden, wobei die Zuleitung in die Trenneinheit und die zumindest eine Rückleitung von der Trenneinheit in verschiedenen Höhen so ausgebildet sind, dass das vom Gaswäscher zugeführte Reinigungsmedium in eine Trennzone zugeleitet wird, während die zumindest eine Leitung von der Trenneinheit zum Gaswäscher in einer Höhe angeordnet ist, so dass angereichertes bzw. in der Trenneinheit abgetrenntes Reinigungsmedium oder ein Teil des Reinigungsmediums oder eine der Flüssigkeiten bei einem Gemisch eines Reinigungsmediums in den Gaswäscher zurückgeführt wird. Vorzugsweise ist zumindest eine Pumpe vorgesehen, mittels der das zurückgeführte und/oder das in die Trenneinheit zufließende Medium umgepumpt wird.

Die Kombi-Mischgasanlage weist vorzugsweise eine Fluidleitung, insbesondere eine Flüssigkeits-Leitung, zwischen der Trenneinheit und der Fermentationseinrichtung auf. Vorzugsweise wird mittels der Fluid- oder Flüssigkeitsleitung eine in der Trenneinheit ausgeschiedene, wässrige Lösung in die Fermentationseinrichtung überführt. Vorzugsweise wird die wässrige Lösung mittels einer Pumpe von der Reinigungseinheit zur Fermentationseinrichtung gefördert. Die wässrige Lösung kann darin gelöste, wasserlösliche Stoffe und/oder Feststoffbestandteile aufweisen, die bei der Reinigung des Verschwelungsgasstroms in der Reinigungseinrichtung abgetrennt bzw. ausgefiltert wurden. Durch vorzugsweise vollständiges Überführen der wässrigen Lösung von der Reinigungseinrichtung in die Fermentationseinrichtung werden die gelösten, wasserlöslichen Stoffe und/oder Feststoffbestandteile 'entsorgt'. Zum Anderen wird aus der Reinigungseinrichtung mit der wässrigen Lösung vorzugsweise Wasser, das sich aufgrund der Kondensation aus dem Verschwelungsgasstrom in der Reinigungseinrichtung anreichert, ebenfalls 'entsorgt' und dient gleichzeitig in der Fermentationseinrichtung als einer der Stoffe zur Anreicherung bzw. Erzeugung des Mischgases in der Fermentationseinrichtung. Bei der vollständigen Überführung der wässrigen Lösung werden mineralische Stoffe in der Fermentationseinrichtung angereichert, so dass die aus der Fermentationseinrichtung nach der Fermentation entnommenen Abfallprodukte (organischer Dünger bzw. Gärreste) neben dem umgesetzten biologischen Dünger auch einen hohen mineralischen Anteil aufweisen. Alternativ oder zusätzlich kann die wässrige Lösung auch aus der Reinigungseinrichtung entnommen werden, so dass die wässrige Lösung als Dünger (mineralhaltiger Dünger) in der Landwirtschaft verwertbar ist.

Vorzugsweise ist das Lösungsmittel wasserunlöslich und eine 'Durchmischung' in einer Mischphase kommt - zumindest in einem Gaswäscher der Gasreinigungsvorrichtung - nur durch aktive Durchmischung zustande. Besonders vorteilhaft wird als Reinigungsmedium in der Gasreinigungseinrichtung, vorzugsweise im Gaswäscher, ein organisches Lösungsmittel eingesetzt. Wie oben bereits erwähnt, kann das Lösungsmittel - abgesehen von den aus dem Verschwelungsgasstrom herausgefilterten Verunreinigungen - in reiner Form vorliegen, vorzugsweise arbeitet die Gasreinigungseinrichtung aber mit einem Gemisch verschiedener Medien, das das organische Lösungsmittel enthält. Das Gemisch ist vorzugsweise das organische Lösungsmittel gemischt mit dem Wasser, das vorzugsweise aus dem Verschwelungsgasstrom kondensiert wurde. Vorzugsweise ist das organische Lösungsmittel organisches Öl, wie Methylester oder Rapsmethylester (RME).

Da das verwendete Fluid in der vorzugsweisen Ausgestaltung bei Trennung zwischen Gaswäscher und Trenneinheit in einem geschlossenen Kreislauf rezykliert wird, wird nach der Erstbefüllung der Reinigungseinrichtung nur eine geringe weitere Zufuhr von Lösungsmittel (organisches und/oder anorganisches) notwendig sein. Beim laufenden Betrieb, bei dem das Lösungsmittel hauptsächlich beim Umsetzen von in den Verschwelungsgasen mitgeführten Teerbestandteilen aufgebraucht wird (bei dem aber auch ein Teil des organischen Lösungsmittels entweder durch die Rückführung in die Verschwelungseinrichtung und/oder die Weiterleitung nach der Austrennung vom Gas in die Fermentationseinrichtung ausgetragen wird), ist der Nachfüllbedarf an Lösungsmittel gering. Es ist daher zu erwarten, dass außer den kohlenstoffhaltigen Ausgangsstoffen, die in die Verschwelungseinrichtung zugeführt werden, und/oder den ggf. rein organischen Ausgangsmaterialien, die der Fermentationseinrichtung zugeführt werden, keine oder nicht nennenswerte, zusätzliche Betriebsstoffe erforderlich sind.

Bei dem Verfahren gemäß Anspruch 11 kommen die oben beschriebenen Elemente bzw. Prozessschritte einzeln oder in beliebiger Kombination zum Einsatz, so dass die oben beschriebenen Merkmale entsprechend einzeln oder in beliebiger Kombination für das Verfahren zutreffen. Sowohl bei der Mischgasanlage als auch beim Verfahren können die unten beschriebenen Elemente bzw. Prozessschritte einzeln oder in beliebiger Kombination hinzugefügt werden.

Beim Reinigungsschritt werden aus den Verschwelungsgasen in der Reinigungseinrichtung die im Gasstrom mitgeführten Teerbestandteile in eine Lösungsmittelphase überfuhrt. Vorteilhaft werden die mineralischen und wasserlöslichen, im Verschwelungsgas mitgeführten Bestandteile in eine wässrige Phase überfuhrt, wodurch der Gasstrom weitgehend gereinigt ist.

Vorzugsweise werden die aus den Verschwelungsgasen entfernten wasserlöslichen Bestandteile und Feststoffe dem Fermenter zugeführt.

Die Gasreinigungseinrichtung zur Reinigung von Gasen aus der Verschwelungseinrichtung kann mit einer Fermentationseinrichtung wie oben und unten beschrieben kombiniert werden. Die Gasreinigungseinrichtung ist dazu ausgelegt, zumindest Teerbestandteile aus einem Gasstrom zu entfernen. Die Gasreinigungseinrichtung weist auf: einen Gaswäscher mit einem Medium zum Aussondern der nicht-gasförmigen Bestandteile aus dem Verschwelungsgasstrom, eine separat vom Gaswäscher angeordnete Trenneinheit, in der sich die aus dem Verschwelungsgasstrom ausgesonderten, nicht-gasförmigen Bestandteile in verschiedene Phasen trennen, eine erste Verbindungsleitung zwischen dem Gaswäscher und der Trenneinheit zum Überführen des durch die Gaswäsche verunreinigten Mediums in eine erste Trennzone der Trenneinheit, und eine zweite Verbindungsleitung zwischen der Trenneinheit und dem Gaswäscher zur Rückführung des durch Phasentrennung in einer zweiten Trennzone der Trenneinheit gereinigten bzw. angereicherten Mediums in den Gaswäscher.

Vorteilhaft ist die Gasreinigungseinrichtung, insbesondere der Gaswäscher, zumindest teilweise mit einem vorzugsweise organischen Lösungsmittel gefüllt ist, insbesondere mit einem organischen Öl, mit Methylester oder mit Rapsmethylester (RME). Vorzugsweise weist die Gasreinigungseinrichtung weiterhin ein, zwei, mehrere oder alle der folgenden Einrichtungen und/oder Leitungen auf: eine Entnahmeeinrichtung oder -leitung zur Entnahme einer durch Phasentrennung in einer dritten Trennzone angereicherten teerhaltigen Lösungsphase aus der Trenneinheit, und/oder eine erste Auslasseinrichtung oder -leitung zur Entnahme einer durch Phasentrennung in einer vierten Trennzone angereicherten wässrigen Lösungsphase, und/oder eine zweite Auslasseinrichtung oder - leitung zur Entnahme einer durch Phasentrennung in einer fünften Trennzone Feststoff-angereicherten Phase.

In Ausgestaltung ist der Gaswäscher ausgelegt zum Lösen der Teerbestandteile und/oder zum Lösen der wasserlöslichen Stoffe und/oder zum Ausfällen der Feststoffbestandteile und/oder zum Kondensieren von Wasserdampf aus den Verschwelungsgasen.

Vorteilhaft trennt sich in der Trenneinheit eine teerhaltige Lösungsmittelphase vom Medium des Gaswäschers und/oder die ausgefällten Feststoffbestandteile setzen sich ab oder fällen aus und/oder es trennt sich kondensiertes Wasser vom Medium und/oder eine wässrige Lösung trennt sich von wasserlöslichen nicht-gasförmigen Bestandteilen vom Medium.

In Ausgestaltung ist die Gasreinigungseinrichtung weiterhin dazu ausgelegt, Feststoffbestandteile und/oder wasserlösliche Stoffe und/oder Wasserdampf aus den Verschwelungsgasen zu entfernen, insbesondere mittels einer Phasentrennung die Feststoffbestandteile und/oder die wasserlöslichen Stoffe und/oder den Wasserdampf aus den Verschwelungsgasen auszusondern.

Vorzugsweise kommunizieren der Gaswäscher und die oder eine Trenneinheit so fluid miteinander, sind insbesondere über die erste und zweite Verbindungsleitungen so miteinander verbunden, dass ein Medium-Fluidkreislauf zwischen Gaswäscher und Trenneinheit hergestellt ist, wobei insbesondere das durch die Gaswäsche verunreinigte Medium dem Gaswäscher entnommen und der Trenneinheit zugeführt wird und gereinigtes Medium der Trenneinheit entnommen und dem Gaswäscher zugeführt wird.

In Ausgestaltung ist der Gaswäscher so ausgestaltet, dass das Gas unten in die flüssige Phase des Reinigungsmediums eingeleitet wird und blasenförmig im Medium aufsteigt. Vorteilhaft sind eine Düseneinrichtung und/oder ein netzartig ausgestalteter Einlass vorgesehen, so dass kleine Blasengrößen in das Reinigungsmedium eintreten. Dies erhöht die Reinigungswirkung und das Kühlen des Gases. Alternativ oder in Kombination mit der Blasenbildung wird das einströmende Gas berieselt, wobei das Reinigungsmedium über eine Flüssigkeitsverteilungseinrichtung in ein Volumen, das vom Gasstrom durchströmt wird, fein verteilt eingesprüht wird. In vorteilhafter Ausgestaltung ist der Gaswäscher ohne Einsprüheinrichtung vorgesehen, so dass kein Einsprühen des (umgewälzten und/oder aus der Trenneinheit rückgeführten) Mediums erfolgt.

Vorzugsweise ist die Verschwelungseinrichtung ein Mehrzonen-Vergaser und eine Vergaseranordnung, bei denen die Verschwelungs- bzw. Vergasungsprozesse in den Zonen steuer- und/oder optimierbar sind. Dies wird wie folgt und am Beschreibungsende angegeben gelöst.

Gemäß diesem weiteren Aspekt ist ein Vergaser zur Vergasung von kohlenstoffhaltigem Material vorgesehen, der auch als Pyrolysator oder Verschwelungseinrichtung bezeichnet werden kann. Im Vergaser werden durch thermische Prozesse Verschwelungsgase erzeugt. Als Ausgangsmaterial, das im Vergaser verschwelt wird, wird kohlenstoffhaltiges Material verwendet, was sowohl organisches Material als auch anorganisches Material einschließen kann. Sowohl das organische Material wie auch das anorganische Material können Abfälle und/oder Rohstoffe/-produkte sein.

Der Vergaser ist in drei (Haupt-)Zonen bzw. (Haupt-)Kammern unterteilt, die in Reihe angeordnet sind und durch jeweils eine Trenneinrichtung voneinander getrennt sind. Die Trenneinrichtungen sind vorzugsweise gasdurchlässig, verhindern jedoch einen unkontrollierten Materialtransport von einer Kammer in die angrenzende Kammer bzw. Zone und verhindern auch, dass die Zonen innerhalb des Vergasers räumlich wandern oder sich verlagern. Die Trenneinrichtungen zwischen zwei benachbarten Kammern bzw. Zonen weisen zumindest eine verschließbare Öffnung bzw. Schleuse auf, durch die durch gesteuertes Öffnen und Schließen kontrolliert Material von der einen in die nächste Kammer (gleichwertig: Zone) transportiert werden kann. Durch den kontrollierten Materialfluss von Kammer zu Kammer kann die in der folgenden (oder vorausgehenden) Kammer vorhandene Materialmenge eingestellt und so der in der Kammer ablaufende Prozess gesteuert und optimiert werden. Der hier angesprochene Materialfluss ist der Feststoffphasen-Materialfluss, der hier vom Gasphasen-Materialfluss zu unterscheiden ist. Vorzugsweise sind die Kammern vertikal zumindest teilweise übereinanderliegend angeordnet und weiter vorzugsweise ist der Festkörper-Materialfluss von oben nach unten, so dass zum Transport die Schwerkraft genutzt werden kann. In Ausgestaltung können die Kammern aber auch nebeneinander (z.B. horizontal nebeneinander) angeordnet sein oder der Materialfluss von unten nach oben gerichtet sein. In diesen Fällen ist zumindest eine Material-Transporteinrichtung vorgesehen, die das Material von Kammer zu Kammer überführt. In Ausgestaltung und bei allen Aspekten der Erfindung ist es nicht notwendig, dass zwischen allen Kammern des Vergasers eine verschließbare Öffnung bzw. Schleuse vorgesehen ist. Besonders im Falle des Feststoffphasen-Materialflusses von oben nach unten kann der Materialtransport schwerkraftbedingt z.B. durch Herunterrieseln des Materials erfolgen.

Die in Feststoffphase-Materialflussrichtung erste Kammer des Vergasers ist eine Trocknungskammer mit einer Beschickungsöffhung zum Trocknen des durch die Beschickungsöffnung zugeführten kohlenstoffhaltigen Materials. Das Trocknen des Materials erfolgt durch die Abwärme einer der nachfolgenden Kammern und/oder durch Abwärme einer dem Vergaser nachfolgenden Prozessstufe (z.B. von einer Gasreinigungseinrichtung und/oder einem Fermenter) und/oder durch das Abgas einer Verbrennungsmaschine oder Heizeinrichtung, die vorzugsweise mittels der Verschwelungsgase oder weiterbehandelten Produkte der Verschwelungsgase des Vergasers betrieben wird. In der Trocknungskammer wird das Material unter Austreibung oder Entzug von Wasser getrocknet, das vorzugsweise als Wasserdampf aus der Trocknungskammer ausgelassen wird. Vorzugsweise wird der Wasserdampf nicht oder nur zu einem geringen Anteil in die anschließende Pyrolysekammer überführt. Ganz besonders vorteilhaft wird der Wasserdampf geregelt in die Brennkammer eingeleitet.

Eine Pyrolysekammer ist der Trocknungskammer nachgeschaltet und das aus der Trocknungskammer zugeführte Material wird zumindest teilweise thermisch durch Pyrolyse umgesetzt (vergast). Dazu wird das in der Trocknungskammer getrocknete Material durch die zumindest eine Öffnung, vorzugsweise durch die zumindest eine verschließbare Öffnung oder Schleuse, in die Pyrolysekammer kontrolliert zugeführt, so dass durch kontrollierte Zufuhr aus der Trocknungskammer und kontrollierten Austrag aus der Pyrolysekammer in die Brennkammer in der Pyrolysekammer eine kontrollierte Materialmenge einstellbar ist. Durch die Materialmenge (und die Verweildauer des Materials in der Pyrolysekammer) ist wiederum der Pyrolyseprozess kontrollierbar.

In der auf die Pyrolysekammer folgende Brennkammer wird das in der Pyrolysekammer thermisch behandelte Material unter Sauerstoffzugabe umgesetzt, vorzugsweise durch einen Oxidations- und Reduktionsprozess. Das in der Pyrolysekammer thermisch behandelte Material wird wiederum durch zumindest eine Öffnung, vorzugsweise durch zumindest eine verschließbare Öffnung oder Schleuse, in die Brennkammer kontrolliert zugeführt. In der Brennkammer findet in einer Brennzone die Verbrennung/Umsetzung statt. Auch hier ist die Menge des am Umsetzungsprozess teilnehmenden Materials durch die kontrollierbare Zufuhr aus der Pyrolysekammer kontrollierbar und somit der Umsetzungsprozess in der Brennzone an sich. Ganz besonders vorteilhaft ist die Brennzone eine Wirbelschichtbrennzone (Wirbelschichtverbrennung (Oxidation/Reduktion)), so dass die Lage der Brennzone in der Kammer noch weiter festgelegt ist, für die inneren Umsetzungsprozesse in der Brennzone kein Wandkontakt vorhanden ist und der Materialkontakt (wie z.B. bei einer Festbettverbrennung) keinen wesentlichen Einfluss auf die Umsetzungsprozesse in der Brennzone hat. Vorteilhaft hat die Brennkammer keine Festbettverbrennungszone und/oder keine Wirbelschicht-Festkörperzusätze (wie Sand), die nicht an den Umsetzungsprozessen teilnehmen.

Aus der Brennkammer werden die Verschwelungsgase entnommen. Vorzugsweise werden die Verschwelungsgase des Vergasers zur Weiterverwertung und/oder -verarbeitung nur aus der Brennkammer entnommen. Wenn hier im Folgenden von 'Verschwelungsgasen' des Vergasers oder Pyrolysators gesprochen wird, so sind damit überwiegend die Umsetzungsprodukte aus dem Oxidations-/Reduktionsprozess der Brennzone gemeint. Jedoch können im Verschwelungsgas auch die Pyrolysegase aus der Pyrolysekammer enthalten sein. Weiterhin enthält der Verschwelungsstrom aus der Brennkammer neben den Verschwelungsgase noch Dampfphasen (z.B. Wasser, Teerdampf, Öle) und Feststoffphasen (z.B. Asche, Holzstaub, Teerpartikel, Teerstaub). Vorzugsweise werden die Bestandteile des Verschwelungsgasstroms einer Nachbehandlung in einer Kombi-Mischgasanlage oder einer Vergaseranordnung unterzogen, so dass die verwertbaren Verschwelungsgase angereichert oder zu noch brauchbarerem Mischgas umgesetzt werden.

Wie oben angeführt, kann das Trocknen in der Trocknungskammer mittels der Abwärme des Vergasers oder einer nachgeschalteten Gasaufbereitungs- und/oder Gasumsetzungseinheit erfolgen. In Ausgestaltung ist eine Abgasleitung an die Trocknungskammer zur Zuführung von Abgas angeschlossen. Mittels des zugeführten Abgases ist die Trocknungskammer gegen Umgebungssauerstoff abgedichtet und/oder das zugeführte kohlenstoffhaltige Material wird damit getrocknet. Vorzugsweise wird zum Trocknen und zur Abgasabdichtung das Abgas einer Verbrennungsmaschine (z.B. BHKW) oder einer Heizeinrichtung eingesetzt. Die Dichtung erfolgt vorzugsweise durch Einleiten des Abgases mit leichtem Überdruck in Bezug auf den Umgebungsdruck des Vergasers und/oder in Bezug auf den Druck in der nachgeschalteten Pyrolysekammer. Vorteilhaft wird dabei der Verbrennungsmotor und/oder der Heizbrenner mittels des Verschwelungsgases des Vergasers oder eines Folgeprodukts des Verschwelungsgases des Vergaser betrieben ist, so dass sich die Energieausbeute der Anlagenkombination erhöht.

In Ausgestaltung ist die Brennkammer über eine Fluidleitung mit der Trocknungskammer verbunden, so dass vorzugsweise Gase aus der Trocknungskammer in die Brennkammer zuführbar sind. Vorzugsweise wird der durch die Trocknung freigesetzte Wasserdampf aus der Trocknungskammer in die Brennkammer zugeführt. Durch den Wasserdampf können die Temperatur der Brennzone und/oder das Reaktionsgleichgewicht der Umsetzungsprozesse in der Brennzone so verschoben werden, dass der Anteil der verwertbaren Verschwelungsgase erhöht wird.

Ganz besonders vorteilhaft ist der Fluidleitung eine Dosiereinrichtung und/oder eine Pumpeinrichtung zugeordnet, mit der Fluidstrom (z.B. Durchflussrate des Wasserdampfs) von der Trocknungskammer zur Brennkammer durch Ansteuerung der Dosiereinrichtung und/oder der Pumpeinrichtung mittels einer Steuereinrichtung des Vergasers steuerbar oder regelbar ist. Damit werden Brenntemperatur und/oder Reaktionsgleichgewicht des Umsetzungsprozesses noch besser steuerbar. In Ausgestaltung ist mittels der Steuereinrichtung der Fluidstrom in Abhängigkeit der Temperatur und/oder des Lambdawertes der Umsetzungsprozesse (Verbrennung) in der Brennkammer einstellbar oder regelbar.

In Ausgestaltung ist das kohlenstoffhaltige Material durch eine gasdichte oder zumindest weitgehend gasdichte Beschickungsöffhung in die Trocknungskammer zuführbar. Wegen der gasdichten Ausgestaltung wird - außer während der Befüllungsphasen - Sauerstoffeintrag in die Trocknungskammer verhindert. Vorteilhaft ist zumindest eine verschließbare Öffnung vorgesehen, die verschließbar ausgebildet ist oder die als Schleuse ausgebildet ist. Vorzugsweise ist der Überdruck oder leichte Überdruck durch das aus der Zuleitung zugeführte Abgas so, dass auch in Befüllungsphasen kein oder kaum Sauerstoff in die Trocknungskammer gelangt.

Vorteilhaft ist der Brennkammer durch eine Sauerstoffleitung oder -öffnung Luft oder Sauerstoff zuführbar, so dass dem Verbrennungsprozess in der Brennzone Sauerstoff zugeführt werden kann. In Ausgestaltung ist die Sauerstoffleitung durch die Trocknungskammer und/oder durch die Pyrolysekammer geführt. Dadurch wird der zugeführte Sauerstoff bzw. die zugeführte Luft vorgewärmt. Vorteilhaft ist der Sauerstoffleitung eine Pumpeinrichtung und/oder eine Dosiereinrichtung zugeordnet ist, so dass der Luft- oder Sauerstoffstrom zur Brennkammer durch Ansteuerung der Dosiereinrichtung und/oder der Pumpeinrichtung mittels einer oder der Steuereinrichtung des Vergasers steuerbar oder regelbar ist. Damit kann der Umsetzungsprozess in der Brennzone optimiert werden. Beispielsweise kann mittels der Steuereinrichtung der Luft- oder Sauerstoffstrom in Abhängigkeit der Temperatur und/oder des Lambdawertes der Verbrennung in der Brennkammer eingestellt oder geregelt werden.

In Ausgestaltung ist ein Betätigungselement vorgesehen, mit dem ein oder mehrere der folgenden betätigbar ist: ein Verschließelement der Öffnung oder Schleuse zwischen der Außenseite des Vergasers und der Trocknungskammer; ein Verschließelement der Öffnung oder Schleuse zwischen der Trocknungskammer und der Pyrolysekammer; und ein Verschließelement der Öffnung oder Schleuse zwischen der Pyrolysekammer und der Brennkammer. In einer besonders bevorzugten Ausgestaltung können mit dem Betätigungselement mehrere Verschließelemente betätigt werden und vorzugsweise ist das Betätigungselement zeitweise von dem oder einem der Verschließelemente der Öffnung oder von der oder einer der Schleusen abkoppelbar, so dass bei Betätigung einer oder mehrerer der anderen Verschließelemente oder Schleusen das abgekoppelte Verschließelement oder die abgekoppelte Schleuse nicht betätigt wird. Besonders vorteilhaft wird mittels des Betätigungselements das Verschließelement der Öffnungen oder Schleusen der Pyrolysekammer und der Brennkammer betätigt, wobei insbesondere das abkoppelbare Verschließelement oder die abkoppelbare Schleuse dasjenige oder diejenige zwischen der Pyrolysekammer und der Brennkammer ist. Ganz besonders vorteilhaft ist das Betätigungselement die Sauerstoffleitung oder ein Stützelement für die Sauerstoffleitung.

Gemäß dem weiteren Aspekt ist eine Vergaseranordnung mit einem Vergaser in Ausgestaltung wie oben beschrieben vorgesehen. Dem Vergaser ist eine Gasreinigungseinrichtung zur Reinigung von Verschwelungsgasen aus dem Vergaser nachgeschaltet. Mittels der Gasreinigungseinrichtung sind Teeranteile aus dem Verschwelungsgas entfernbar. Teeranteile im Verschwelungsgasstrom können dampfförmige Teere sein, mitgeführte Teerpartikel oder an anderen Festkörperpartikeln (Asche, Holzkohle) angelagerte Teere. Zwischen der Gasreinigungseinrichtung und dem Vergaser ist eine Rückführleitung angeordnet ist mit der die in der Gasreinigungseinrichtung entfernten Teeranteile und/oder die in der Gasreinigungseinrichtung umgesetzten Teeranteile in den Vergaser zurückführbar sind. Vorteilhaft sind dabei über die Rückführleitung die entfernten Teeranteile und/oder die umgesetzten Teeranteile in die Pyrolysekammer oder die Brennkammer zurückführbar. Beispielsweise erfolgt die Rückführung durch Einleiten oder Einsprühen in die Brennkammer.

In Ausgestaltung weist die Gasreinigungseinrichtung einen Gaswäscher auf, der vorzugsweise zumindest teilweise mit einem organischen Lösungsmittel gefüllt ist, insbesondere mit einem organischen Öl, mit Methylester, Glycerin und/oder mit Rapsmethylester (RME). Mit diesem Lösungsmittel werden die Teeranteile (Teerbestandteile) aufgelöst und in eine flüssige Lösungsmittelphase überführt. Die umgesetzten Teeranteile liegen dann zumindest teilweise in einer niederkettigeren Form vor, die z.B. in der Brennzone der Brennkammer mit größerer Rate in CO, CH₄ oder CO₂ umgesetzt wird.

Die Teerbestandteile sind kohlenstoffhaltig und können daher ebenfalls als Ausgangsmaterial zur Erzeugung von Methan und CO₂ sowie anderen kohlenstoffhaltigen Gasen in der Verschwelungseinrichtung umgesetzt werden. Es ist daher durch die Rückführung der Teerbestandteile in den Verschwelungsprozess nicht notwendig, den Teer als Abfallmaterial einer Sondermüllbehandlung zuzuführen, sondern es wird so lange im Kreislauf zwischen Verschwelungseinrichtung und Gasreinigungseinrichtung durchlaufen lassen, bis eine im Wesentlichen vollständige oder vollständige Umsetzung in zur Mischgaserzeugung verwertbare Verschwelungsgaskomponenten stattgefunden hat.

Vorzugsweise weist die Gasreinigungseinrichtung eine vorzugsweise separat vom Gaswäscher angeordnete Trenneinheit auf, in der sich die aus dem Verschwelungsgasstrom ausgesonderten, nicht-gasförmigen Bestandteile in verschiedene Phasen trennen, wobei sich insbesondere eine teerhaltige Lösungsmittelphase vom Medium des Gaswäschers trennt und/oder in der sich die ausgefällten Feststoffbestandteile absetzen oder ausfällen und/oder sich kondensiertes Wasser vom Medium trennt und/oder sich eine wässrige Lösung von wasserlöslichen nicht-gasförmigen Bestandteilen vom Medium trennt.

Im Verschwelungsgasstrom von der Verschwelungseinrichtung zur Gasreinigungseinrichtung werden als gasförmige und/oder dampfförmige und/oder staub- bzw. feststoffförmige Verschwelungsprodukte ein oder mehrere der folgenden Verunreinigungen mitgeführt: Wasserdampf, Asche, Teer in Partikelform (als Kondensat) oder dampfförmig oder dergleichen. Die Asche oder Feststoffpartikel können organischer oder anorganischer Natur sein (beispielsweise mineralische oder kohlenstoffhaltige Asche oder Feststoffpartikel).

Mittels der Gasreinigungseinrichtung werden die Verunreinigungen vollständig oder weitgehend aus dem Verschwelungsgasstrom entfernt. Verschwelungsgas umfasst hier hauptsächlich H₂, Methan, CO und CO₂, kann aber auch andere Verschwelungsgase enthalten.

Vorteilhaft werden die gereinigten Verschwelungsgase aus der Gasreinigungseinrichtung einer nachgeschalteten Fermentationseinrichtung zugeführt, um diese in ein methan- und/oder Wasserstoff-angereichertes Gas ('Biogas') umzuwandeln. Vorzugsweise wird die Fermentationseinrichtung neben dem gereinigten Verschwelungsgas mit kohlenstoffhaltigem Material beschickt (oder anderen üblichen Einsatzstoffen), das dort durch einen Fermentationsprozess unterstützt mit den gereinigten Verschwelungsgasen zu dem H₂- und/oder Methan-angereicherten Gas umgesetzt wird. Vorzugsweise ist das der Fermentationseinrichtung zugeführte Material ausschließlich organisches Material (biologische Abfälle wie land-/forstwirtschaftliche Erzeugnisse). Vorzugsweise wird die Zusammensetzung der Verschwelungsgase in der Fermentation durch die bakterielle Umsetzung so geändert, dass sich der Anteil von CH₄ zu Lasten des Anteils an H₂ und CO erhöht.

Gemäß einer vorteilhaften Ausgestaltung ist die Gasreinigungseinrichtung weiterhin dazu ausgelegt, eine oder alle der folgenden Verunreinigungsbestandteile aus dem Verschwelungsgasstrom zu entfernen oder herauszufiltern: Lösungsmittel-lösliche Stoffe (z.B. Teer wie oben beschrieben); Feststoffbestandteile (beispielsweise Asche), die im Verschwelungsgasstrom von der Verschwelungseinrichtung mitgeführt werden; wasserlösliche Stoffe, die ebenfalls im Verschwelungsgasstrom mitgeführt werden, wie beispielsweise wasserlösliche Gase, wasserlösliche Feststoffbestandteile oder Flüssigkeitströpfchen oder dampfförmige Phasen aus der Zersetzung in der Verschwelungseinrichtung; Wasserdampf der vorzugsweise aufgrund der Abkühlung des Verschwelungsgasstroms in der Gasreinigungseinrichtung auskondensiert.

Vorzugsweise ist die Gasreinigungseinrichtung dazu ausgelegt, die Temperatur des Verschwelungsgasstromes herunter zu kühlen, so dass die kondensierbaren Gase oder die Dämpfe in der Gasreinigungseinrichtung kondensieren und so aus dem Verschwelungsgasstrom entfernt werden. Vorzugsweise wird die aus der Abkühlung des Verschwelungsgasstroms in der Gasreinigungseinrichtung freigesetzte Wärme als Prozesswärme genutzt und innerhalb der Kombi-Mischgasanlage weiterverwendet. Beispielsweise wird die in der Gasreinigungseinrichtung gewonnene Prozesswärme dazu verwendet, das kohlenstoffhaltige Ausgangsmaterial, das der Verschwelungseinrichtung zugeführt wird oder ein biologisches Ausgangsmaterial, das der Fermentationseinrichtung zur Umsetzung in Mischgas zugeführt wird, vorzuwärmen und/oder zu trocknen.

Gemäß einer vorteilhaften Ausgestaltung werden in der Gasreinigungseinrichtung leicht kondensierbare Gase und/oder dampfförmige Stoffe durch Abkühlung kondensiert (siehe oben) und das Kondensat wird gleichzeitig als ausschließliches Reinigungsmedium in der Gasreinigungseinrichtung verwendet oder als Bestandteil eines Gemisches von Reinigungsmedien oder verschiedener Phasen von Reinigungsmedien in der Gasreinigungseinrichtung verwendet. Vorzugsweise ist die durch Abkühlung kondensierte Flüssigkeit ausschließlich Wasser oder überwiegend Wasser, welches dann zur Lösung von wasserlöslichen Bestandteilen aus dem Verschwelungsgasstrom verwendet wird. Beispielsweise lassen sich Salze bzw. mineralische Bestandteile im Wasser lösen, so dass diese aus dem Verschwelungsgasstrom in Lösung im Wasser übergehen.

Ein Ausführungsbeispiel der Erfindung ist in den nachfolgenden Figuren dargestellt, die zeigen:
- Fig. 1: eine Kombi-Biogasanlage in schematischer Darstellung mit einem Vergaser, einem Gasreiniger und einem Fermenter, und
- Fig. 2: den Vergaser von Fig. 1 in detaillierterer Blockdarstellung.

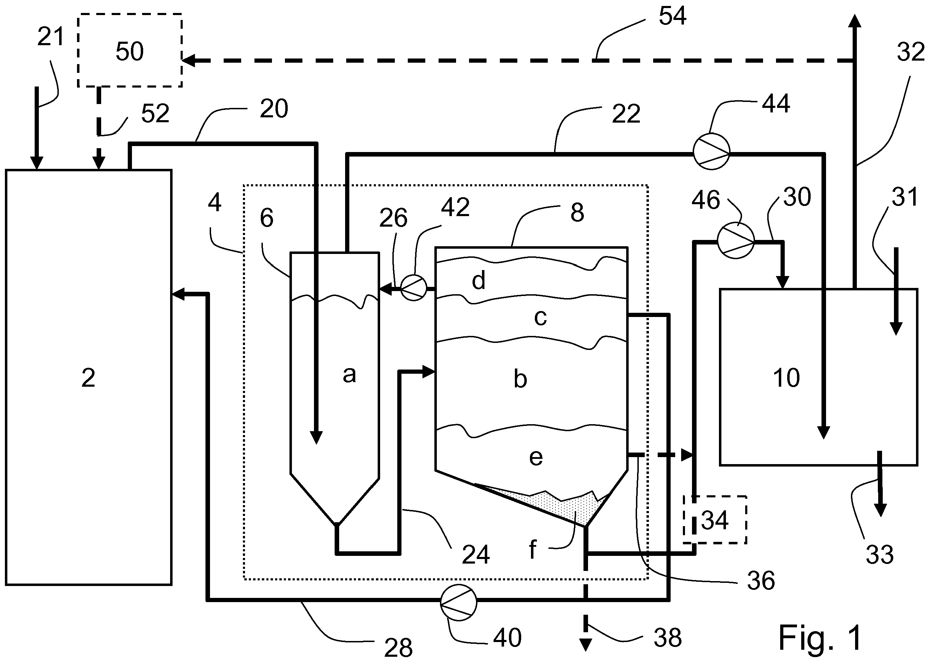

Die in Fig. 1 dargestellte Kombi-Biogasanlage weist die Hauptkomponenten Pyrolysator bzw. Vergaser 2, Gasreiniger 4 und Fermenter 10 auf. Im Pyrolysator 2 wird kohlenstoffhaltiges Ausgangsmaterial (hier zugeführt in Form einer Materialzufuhr 21) thermisch zersetzt. Das kohlenstoffhaltige Ausgangsmaterial kann biologisches Material sein, oder nicht-biologisches, kohlenstoffhaltiges Material, wie Plastikabfälle oder sonstige Mineralöl-basierende Abfälle, sowie ein Gemisch hiervon. Dies hat den Vorteil, dass die Kombi-Biogasanlage sowohl landwirtschaftliche als auch nicht-landwirtschaftliche Abfälle thermisch verwerten kann.

Im Pyrolysator 2 entstehen Verschwelungsgase, die prozessbedingt nicht nur die verwertbaren Gase wie H₂, CO, CO₂ und CH₄ enthalten, sondern auch in dieser Form nicht-verwertbare Bestandteile wie Asche, Dämpfe (einschließlich Wasserdampf), Ruß und Teerpartikel. Das Verschwelungsgas plus die Verunreinigungsbestandteile werden vom Pyrolysator 2 über eine Verschwelungsgasleitung 20 in einen Gaswäscher 6 des Gasreinigers 4 zugeführt. In weiterer Ausgestaltung wird der ungefilterte Verschwelungsgasstrom vor dem Einleiten in den Gasreiniger 4 zunächst einer Partikelabscheidung zugeführt, beispielsweise in einem Zyklonabscheider. Im Gaswäscher 6 werden das Verschwelungsgas und die mitgeführten Verunreinigungsbestandteile in eine Waschlösung a geleitet und vorzugsweise mit dieser durch hydraulische oder mechanische Betätigung durchmischt. Beispielsweise ist im Gaswäscher ein Rührwerk vorgesehen oder es sind nicht dargestellte Umwälzleitungen, die eine intensive Durchmischung von Verschwelungsgas und Waschlösung bewirken, vorgesehen.

Im Gaswäscher 6 kommt es zu einer Abkühlung des Verschwelungsgases durch den Kontakt mit der Waschlösung a und die dabei anfallende Prozesswärme kann der Waschlösung a wieder entzogen und beispielsweise zum Trocknen der kohlenstoffhaltigen Ausgangsmaterialien 21 für den Verschwelungsprozess oder zum Trocknen der vorzugsweise biologischen Ausgangsstoffe 31 des Fermenters 10 oder für andere Prozesse verwendet werden. Vorzugsweise wird der Verschwelungsgasstrom im Gasreiniger 4, insbesondere im Gaswäscher, auf unter 100° C abgekühlt, so dass der Wasserdampf im Verschwelungsgasstrom kondensiert. Die im Gaswäscher 6 gereinigten Verschwelungsgase werden über eine Gasleitung 22 in den Fermenter 10 eingeleitet. Vorzugsweise ist eine Gaspumpe 44 vorgesehen, mit der am Gaswäscher 6 ein Unterdruck zum Absaugen der Gase erzeugt wird, wobei sich der Unterdruck über die Verschwelungsgasleitung 20 vorzugsweise fortsetzt, so dass mittels der Gaspumpe 44 auch die Verschwelungsgase aus dem Pyrolysator 2 abgesaugt werden.

Über eine Ablaufleitung 24, die vorzugsweise im unteren Bereich oder am untersten Bodenbereich des Gaswäschers 6 angeordnet ist und so auch schwerkraftbedingte Ausfüllprodukte abführt, wird die mit Verunreinigungen angereicherte Waschlösung a in einen Trennbehälter 8 des Gasreinigers 4 überführt. Der Trennbehälter 8 ist vorzugsweise so dimensioniert und eingerichtet, dass die zugeführte, mit Verunreinigungsbestandteilen beladene Waschlösung b nur einer geringen Strömung bzw. Verwirbelung unterliegt, so dass sich aus der belasteten Waschlösung b schwerkraftbedingt verschiedene, untereinander nicht-lösliche Flüssig- bzw. Feststoffphasen ausbilden. Die belastete Waschlösung b ist so wie sie vom Gaswäscher 6 zugeführt wird, eine Mischung aus Feststoffen bzw. Feststoffpartikeln, Wasser, einer wässrigen Lösung mit wasserlöslichen, herausgefilterten Bestandteilen, Lösungsmittel und einer flüssigen Lösungsphase mit im Lösungsmittel gelösten Bestandteilen. Diese trennen sich innerhalb des Trennbehälters 8 auf. Die Feststoffe sinken schwerkraftbedingt nach unten ab und setzen sich am Boden ab bzw. sie scheiden sich dort als eine Schichtung von Wasser mit sehr hohem Feststoffanteil aus, wodurch sich eine Feststoff-angereicherte Phase f bildet. Darüber ist die wässrige Lösungsphase e bzw. Wasser, welches sich im Gaswäscher 6 durch Kondensation von im Verschwelungsgasstrom mitgeführten Wasserdampf gebildet hat. Oberhalb der belasteten Waschlösung b bildet sich eine Lösungsphase c des organischen Lösungsmittels, in dem die teerhaltigen Verunreinigungsbestandteile aufgelöst sind. Über der teerhaltigen Lösungsphase c bildet sich das angereicherte organische Lösungsmittel d mit vermindertem Teeranteil.

Wenn hier von Trennung und 'Schichtung' gesprochen wird, so bedeutet dies nicht, dass sich innerhalb des Trennbehälters 8 scharfe Phasengrenzen bilden müssen, sondern es sind in der Regel fließende Übergänge zwischen den Schichtungen, zwischen denen sich die Dichte bzw. Konzentration graduell ändert. Vor allem in Hinsicht darauf, dass im Trennbehälter keine statische, dauerhafte Trennung möglich ist, sondern aufgrund der Umwälzung zwischen Wäscher 6 und Trennbehälter 8 stets eine dynamische Grenze verläuft. Die Schichtung ist auch von daher dynamisch, da beispielsweise durch Verunreinigungen bei Veränderung des Ausgangsmaterials im Pyrolysator 2, durch Temperaturänderungen oder durch diskontinuierliches Entnehmen oder Zuführen von Flüssigkeit eine Verschiebung der Höhenlage der Schichtung erfolgt. Es ist darauf hinzuweisen, dass auch für eine Anpassung der Kombi-Biogasanlage an unterschiedliche Größen, Prozessgeschwindigkeiten und/oder Ausgangsmaterialien die Zone bzw. die Höhe im Behälter 8, in der Flüssigkeiten zugeführt oder entnommen werden, in Abhängigkeit der Gegebenheiten angepasst werden müssen. Die Höhe der Zu- oder Ableitung kann beispielsweise prozessbedingt verändert werden oder sie kann so dimensioniert werden, dass nach der individuellen Anpassung auf die Standardprozessbedingungen die Entnahme der gewünschten Phase in der entsprechenden Höhe aus dem Behälter 8 im Wesentlichen oder die meiste Betriebsdauer über gewährleistet ist.

Aus dem Trennbehälter 8 wird die Phase, in der sich das organische Lösungsmittel d anreichert über eine Lösungsmittelleitung 26 entnommen und dem Gaswäscher 6 zugeführt. Vorzugsweise wird die Zirkulation aus Waschlösung a und organisches Lösungsmittel d durch eine Umwälzpumpe 42 unterstützt, die vorzugsweise in der Lösungsmittelleitung 26 angeordnet oder dieser zugeordnet ist, so dass aus dem Behälter 8 das organische Lösungsmittel d entnommen und dem Wäscher zugeführt wird.

Die wässrige Phasen b, e und f bestehen aus mineralischen Aschepartikeln, organischen Fettsäuren, Aldehyden, Ketonen, Phenolen und Stickstoff- und Schwefel-haltigen Verbindungen, die in dieser Zusammensetzung weder als organischer Dünger zugelassen ist, noch als Abwasser einer kommunalen Kläranlage zugeführt werden kann. Diese wässrigen (Kondensat)Phasen würden bei herkömmlichen Biogasanlagen aufwändig aufgearbeitet werden müssen. In der erfindungsgemäßen Kombi-Biogasanlage werden die Phasen e und f (zu einen vorzugsweise geringen Anteil auch die Phase b) über die Leitung 30 dem Biogasfermenter 10 zugeführt. Die Anaerobflora des Fermenters ist bei den Fermentertemperaturen um 40°C in kurzer Zeit in der Lage, die Inhaltsstoffe nicht nur abzubauen, sondern darüber hinaus aus diesen Stoffen noch Methan (CH₄) zu produzieren.

In vorteilhaften Ausgestaltungen wird die Zufuhr der Phasen e und/oder f wie folgt vorgenommen: Vorzugsweise am unteren Ende des Behälters 8 ist eine Absetzleitung 30 angesetzt, mittels der die wässrige Lösungsphase angereichert mit den Feststoffen f aus dem Trennbehälter 8 entnommen und dem Fermenter 10 zugeführt wird. Mit der Feststoff-angereicherten Phase f wird einerseits das für den Fermentationsprozess benötigte Wasser dem Fermenter 10 zugeführt und andererseits werden die Feststoffe, die in der Regel mineralische Stoffe sind, aus dem Trennbehälter 8 'entsorgt'. Durch die Zufuhr der Feststoff-angereicherten Phase f in den Fermenter 10 werden dort die Abfallprodukte um mineralische Substanzen angereichert, so dass dem Fermenter 10 als ein Endprodukt eine mineralisch/organisch-angereichte Lösung entnommen werden kann. Alternativ oder im Mischbetrieb - je nach Anforderung - kann die Feststoff-angereicherte Phase f auch über eine Feststoffleitung 38 entnommen werden, wobei die Feststoff-angereicherte Phase f nach weiterer Aufbereitung beispielsweise als (mineralhaltiger) Dünger verwendet werden kann. In diesem Fall wird dann über die optionale Leitung 36 für wässrige Lösung die wässrige Lösungsphase e aus dem Trennbehälter 8 entnommen und weiter über die Absetzleitung 30 dem Fermenter 10 zugeführt. In der wässrigen Lösungsphase e sind neben dem Wasser wasserlösliche Bestandteile, die aus dem Verschwelungsgasstrom vom Pyrolysator 2 ausgefiltert wurden, enthalten.

Als weitere Alternative können die Feststoffe, die sich in der Feststoff-angereicherten Phase f angereichert haben, mittels eines optionalen Feststofffilters 34 ausgefiltert werden, so dass über die Absetzleitung 30 wiederum nur oder im Wesentlichen nur die wässrige Lösungsphase e dem Fermenter 10 zugeführt wird. Vorzugsweise wird das Entnehmen der Feststoff-angereicherten Phase f und/oder der wässrigen Lösungsphase e durch eine Pumpe 46 für wässrige Lösung unterstützt.

Mittels einer Teerlösungsleitung 28 wird aus dem Trennbehälter 8 die teerhaltige Lösungsphase c in der Höhe entnommen, in der sich diese durch den Absetzprozess anreichert, und wird dem Pyrolysator 2 zugeführt. Vorzugsweise wird die Entnahme der teerhaltigen Lösungsphase c durch eine Teerlösungspumpe 40 unterstützt. Durch das Rückführen der teerhaltigen Lösungsphase c in den Pyrolysator 2 ergibt sich für die Teer- bzw. teerhaltigen Produkte ein Kreislauf zwischen Pyrolysator 2 und Gasreiniger 4 mit dem Ergebnis, dass der im Teer gebundene Kohlenstoff nicht in den Fermenter 10 überführt wird und stattdessen durch die wiederholte Behandlung im Pyrolysator 2 umgesetzt (beispielsweise gecrackt) wird, bis der Kohlenstoff in gasförmiger Form (z.B. CH₄, CO₂, CO) vorliegt. Dies erhöht einerseits die Ausbeute an kohlenstoffhaltigem Gas und vermindert auf der anderen Seite die Menge der Teerprodukte, die als Sondermüll entsorgt werden müssen oder die möglicherweise den Fermentationsprozess im Fermenter 10 ungünstig beeinflussen bzw. abtöten. Vorzugsweise hat die teerhaltige Lösungsphase c ihren Sättigungspunkt erreicht. Vorzugsweise wird die dem Pyrolysator 2 zugeführte teerhaltige Lösungsphase c mittels eines Einspritzmechanismus eingespritzt oder mit dem Ausgangsmaterial des Pyrolysators vermischt und der Vergasungsreaktion zugeführt. In der Oxidationszone des Pyrolysators werden die höheren molekularen C-Verbindungen (Teere) thermisch gecrackt.

In der Kombi-Biogasanlage werden dem Fermenter 10 die wässrige Lösungsphase e und/oder die Feststoffangereicherte Phase f über die Leitung 30 zugeführt, das gereinigte Verschwelungsgas über die Leitung 22. Vorzugsweise wird dem Fermenter über eine Biomassezuführung 31 Biomasse zugeführt. Alternativ kann der Fermenter auch eine methanogene Fermentation durchführen, wie sie beispielsweise für den Fermenter in der EP 1 118 671 A1 beschrieben ist. Das durch den Fermentationsprozess im Behälter 10 entstandene Methangas (Biogas) vermischt sich im Kopfraum des Fermenters mit dem über die Leitung 22 eingetragenen und nicht zu Methan metabolisierten Schwelgas (Produktgas) zu einem Mischgas, das über eine Leitung 32 entnommen wird. Das durch den Fermentationsprozess mit brennbaren Gasen angereicherte Mischgas (Biogas) wird über eine Biogasleitung 32 entnommen. Beispielsweise wird das Mischgasgas oder ein Teil des Mischgases einem Blockheizkraftwerk 50 oder einer anderen gasbetriebenen Einrichtung über eine Leitung 54 zugeführt.

Vorzugsweise werden die Abgase des Blockheizkraftwerkes 50 bzw. der gasgetriebenen Anlage über eine Abgasleitung 52 zumindest teilweise in den Pyrolysator 2 zugeführt, so dass der Sauerstoffanteil während des thermischen Prozesses im Pyrolysator reduziert und somit auch im Pyrolysator 2 bereits eine Verschiebung des Gleichgewichts in Richtung höherem Methananteil zu Lasten des CO im CO2-Anteils verschoben wird.

Als Endprodukt wird dem Fermenter 10 über einen Austrag 33 Gärrest entnommen, der als organischer Flüssigdünger der Düngemittelverordnung entspricht und somit landwirtschaftlich verwertet werden kann. Falls die Feststoff-angereicherte Phase f und/oder die Phase e ebenfalls in den Fermenter 10 zugeführt wird (s.o.) ist der Gärrest noch mit mineralischen Endprodukten angereichert.

Der Inhalt des Gaswäschers 6 umfasst bei dem beschriebenen Ausführungsbeispiel ein Gemisch als Waschlösung a aus dem organischen Lösungsmittel d und Wasser, das durch die Abkühlung des Verbrennungsgasstroms und durch die Kondensation des Wasserdampfes gewonnen wurde. Im organischen Lösungsmittel und Wasser lösen sich durch den Kontakt mit dem ungereinigten Verschwelungsgasstrom die wasserlöslichen und mittels des organischen Lösungsmittels löslichen Verunreinigungsbestandteile im Flüssigkeitsgemisch der Waschlösung a auf. Weiterhin werden die Schwebstoffe wie Asche, Teerpartikel und dergleichen in der Waschlösung a zurückgehalten. Die belastete Waschlösung b wird in den Trennbehälter 8 überführt und dort schwerkraftbedingt in verschiedene Höhenzonen aufgetrennt. Alternativ kann anstelle der Schwerkraft auch eine Phasentrennung mittels mechanisch wirkender Schwerkraftdekanter zum Einsatz kommen, beispielsweise durch Vorsehen eines Zyklons oder einer Schleuder.

Das nach der Trennung wiederum angereicherte (also gereinigte) organische Lösungsmittel d wird zurückgeführt, während das Wasser vorzugsweise dem Fermenter 10 zugeführt wird. Alternativ oder zusätzlich kann das Wasser bzw. die wässrige Lösungsphase e auch dem Pyrolysator 2 gesteuert zugeführt werden, wobei das gesteuert zugeführte Wasser bzw. die wässrige Lösung e zum Abkühlen des Verbrennungsprozesses und damit zur Temperaturregelung im Pyrolysator 2 verwendet werden kann.

Vorzugsweise umfassen die biologischen Ausgangsstoffe für den Pyrolysator 2 und/oder für den Fermenter 10 nachwachsende Rohstoffe und/oder organische Abfälle. Vorzugsweise werden dem Pyrolysator 2 solche organischen Rohstoffe zugeführt, die für eine Vergärung in einer Biogasanlage ungeeignet sind.

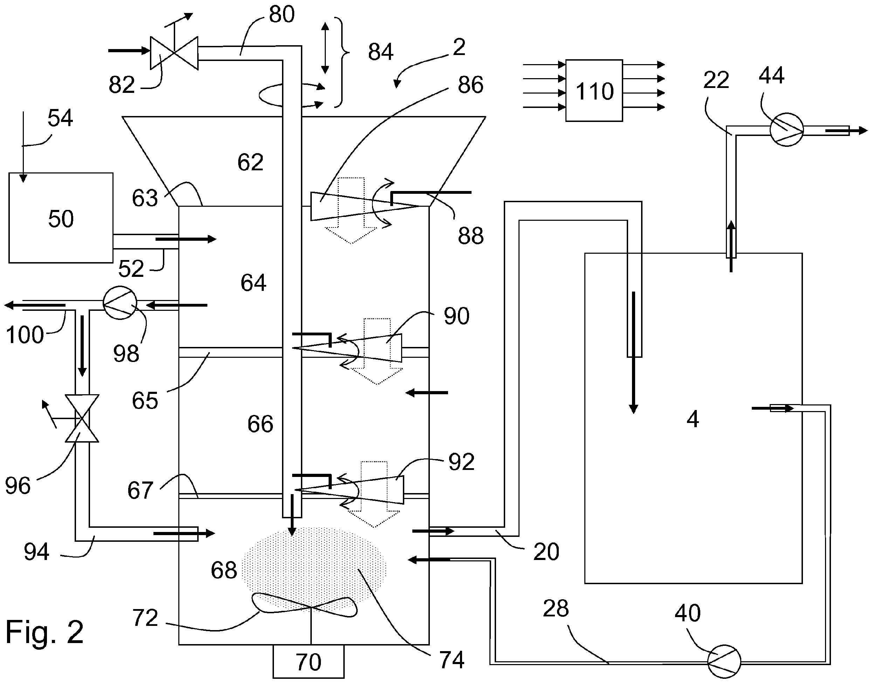

Fig. 2 zeigt den Pyrolysator 2 (Vergaser) in schematischer Detailansicht. Im oberen Bereich hat der Pyrolysator eine Zuführung 62 zur Zuführung des organischen oder anorganischen, kohlenstoffhaltigen Materials. Getrennt durch eine erste Trennwand 63 folgt in Hinsicht auf den Festphasen-Materialfluss (siehe Blockpfeile zwischen den Zonen) eine Trockungskammer 64 in der das Material vorgewärmt und dadurch getrocknet wird. Vorzugsweise liegt die Temperatur in der Trocknungskammer bei 50 bis 60°C. Nach der Trocknungskammer 64 folgt eine Pyrolysekammer 66, die von der Trocknungskammer durch eine zweite Trennwand 65 getrennt ist. In der Pyrolysekammer ist die Temperatur so hoch, dass das in der Trocknungskammer vorgetrocknete Material zumindest teilweise pyrolysiert bzw. verschwelt wird. Vorzugsweise liegt die Temperatur in der Pyrolysekammer um 600°C. An die Pyrolysekammer schließt sich eine Brennkammer 68 an, die von der Pyrolysekammer durch eine dritte Trennwand 67 getrennt ist.

Die erste Trennwand 63 hat eine erste Schleuse 86, die durch einen Aktuator 88 betätigbar ist, um eine Öffnung in der Trennwand zu öffnen und zu verschließen. Bei geöffneter Schleuse 86 fällt das Beschickungsmaterial von der Zuführung 62 in die Trocknungskammer 64. Bei geschlossener Schleuse 86 verschließt die Trennwand 63 die Trocknungskammer (weitgehend) luftdicht bzw. gasdicht gegen die Zufuhrseite 62, so dass keine Umgebungsluft in die Trocknungskammer gelangt.

Zwischen der Trocknungskammer 64 und der Pyrolysekammer 66 ist die zweite Trennwand 65 mit einer zweiten Schleuse 90 angeordnet. Bei geschlossener zweiter Schleuse 90 wird durch die zweite Trennwand 65 ein Materialtransport von der Trocknungskammer zur Pyrolysekammer verhindert. Jedoch ermöglichen die Trennwand 65 und die geschlossene zweite Schleuse 90 einen Druckausgleich zwischen den beiden Kammern 64/66 - also eine Gasströmung zwischen den Kammern. Da einerseits von der Brennkammer 68 über die Pyrolysekammer tendenziell eher ein Unterdruck an der Trocknungskammer anliegt und andererseits in die Trocknungskammer Abgas von dem Blockheizkraftwerk 50 eingeleitet wird und daher ein leichter Überdruck in der Trocknungskammer vorhanden ist, ist die Gasströmung von der Trocknungskammer in die Pyrolysekammer gerichtet.

Zwischen der Pyrolysekammer 66 und der Brennkammer 68 ist die dritte Trennwand 67 mit einer dritten Schleuse 92 angeordnet. Bei geschlossener dritter Schleuse 92 wird durch die dritte Trennwand 67 ein Materialtransport von der Pyrolysekammer zur Brennkammer verhindert. Jedoch ermöglichen die Trennwand 67 und die geschlossene dritte Schleuse 92 einen Druckausgleich zwischen den beiden Kammern 66/68 - also eine Gasströmung zwischen den Kammern. Da durch die Gaspumpe 44 ein Unterdruck über den Gasreiniger 4 und die Leitungen 20, 22 an der Ansaugöffnung der Verschwelgasleitung 20 anliegt und somit ein leichter Unterdruck in der Brennkammer 68, wird das Verschwelgas der Pyrolysekammer 66 in die Brennkammer abgesaugt.

In einer hier nicht dargestellten Ausgestaltung sind die zweite und/oder die dritte Schleuse 90, 92 nicht vorgesehen. Anstelle der zweiten und/oder dritten Schleuse weist die zweite und/oder dritte Trennwand 65, 67 eine oder mehrere Öffnungen und/oder ein oder mehrere Gittereinsätze auf, durch die der Festphasen-Materialfluss von der Trocknungskammer 64 zur Pyrolysekammer und/oder von der Pyrolysekammer 66 zur Brennkammer 68 erfolgt, beispielsweise durch das schwerkraftunterstützte Herunterrieseln des Materials von einer Kammer zur nächsten.

Der Brennkammer wird durch eine Sauerstoffleitung 80 Umgebungsluft einschließlich des darin enthaltenen Sauerstoffs zugeführt. Normalerweise wird der Sauerstoff durch die Leitung 80 in Abhängigkeit des in der Brennkammer vorhandenen Unterdrucks angesaugt, wobei der Unterdruck wiederum auf der Saugwirkung der Gaspumpe 44 beruht. D.h. die Umgebungsluft strömt nur durch den Strömungswiderstand der Leitung 80 begrenzt in die Kammer 68. In Ausgestaltung und wie dargestellt, kann jedoch ein Schieber oder ein Regelventil 82 vorgesehen sein, mit dem die pro Zeiteinheit zugeführte Sauerstoffmenge geregelt wird. Beispielsweise kann die Zusammensetzung des Verschwelungsgases z.B. in der Verschwelungsgasleitung 20 gemessen werden und mit einer Steuereinheit 110 der Zufluss von Umgebungsluft mittels des Regelventils 82 geregelt werden. Beispielsweise wird im Verschwelungsgas der Lambdawert bestimmt und dieser durch Einstellung der zufließenden Sauerstoffmenge eingeregelt.

Die Sauerstoffleitung 80 ist durch die Trocknungskammer 64 und die Pyrolysekammer 66 in die Brennkammer 68 geführt, so dass sich die zugeführte Umgebungsluft bis zum Eintritt in die Kammer 68 aufheizt. Vorzugsweise wirkt die Sauerstoffleitung 80 als Betätigungselement zum Öffnen und Schließen der zweiten und dritten Schleuse 90, 92. Dazu ist der Leitung 80 ein Manipulator 84 zugeordnet, mittels dessen die Leitung 80 eine Schwenkbewegung und eine Längsbewegung (hier Vertikalbewegung) ausführt. Durch das Schwenken werden die Schleusen 90, 92 geöffnet und geschlossen. Durch die Längsbewegung kann die Leitung 80 von der dritten Schleuse 92 entkoppelt werden, so dass durch das Schwenken nur noch die zweite Schleuse 90 geöffnet und geschlossen wird, während die dritte Schleuse 92 in ihrer geschlossenen Stellung verweilt. In Ausgestaltung kann eine Wechselmechanik vorgesehen sein, so dass mittels der Leitung 80 entweder die eine oder die andere Schleuse 90, 92 unabhängig geschlossen und geöffnet werden kann. Vorzugsweise sind die Schleusen 88, 90 und/oder 92 so ausgestaltet, dass die Trennwände 63, 65 und/oder 67 runde plattenförmige Zwischenböden mit jeweils einer oder mehreren Öffnungen sind und die beweglichen Schleusen 88, 90 und/oder 92 drehbare runde Platten mit jeweils einer oder mehreren Öffnungen sind. Wenn bei der Drehung oder beim Schwenken mittels der Leitung 80 die Öffnungen zur Deckung kommen, kann das Material von der oberen Kammer in die daruntergelegene Kammer herunterrieseln.

Die Brennkammer 68 ist vorzugsweise als Wirbelschichtbrennkammer ausgebildet, in der der Oxidationsprozess und der Reduktionsprozess in einer Brennzone 74 (Wirbelbett) stattfinden. Dazu wird das von der Pyrolysekammer in die Brennkammer gespeiste, vorverschwelte Material durch ein Gebläse 72 in der Brennzone in Schwebe gehalten. Der Oxidations- und Reduktionsvorgang arbeitet ohne Festbett und vorzugsweise ohne nichtreaktive Festkörper, d.h. es kommen keine Feststoffe zum Einsatz, die am Verbrennungsprozess nicht teilnehmen - wie beispielsweise Sand, der durch das Gebläse 72 oder durch einen zugeführten Umgebungsluftstrom in der Schwebe gehalten werden muss. D.h. der Prozess wird ausschließlich durch das von der Pyrolysekammer zugeführte, zu verbrennende Material aufrechterhalten und nur die Verschwelungsgase und ggf. Ascherückstände werden aus der Brennkammer herausgeführt.

Das Gebläse 72 wird von einem Gebläsemotor 70 angetrieben. Eine nicht dargestellte Messanordnung misst die Leistungsaufnahme des Motors 70. Anhand der Leistungsaufnahme wird mittels der Steuereinrichtung 110 bestimmt, ob weiteres Material für den Verbrennungsvorgang aus der Pyrolysekammer 66 in die Brennkammer 68 zugeführt werden muss. Ist die Leistungsaufnahme gering, kann daraus geschlossen werden, dass zu wenig Feststoffmaterial in der Brennzone vorhanden ist und es wird durch Öffnen der Schleuse 92 Material aus der Pyrolysekammer zugeführt. Ist die Leistungsaufnahme in einem vorgegebenen Bereich, so ist genügend brennbares Material für eine optimale Verbrennung vorhanden und die Schleuse 92 kann geschlossen werden.

Bei eingefahrenem Verschwelungsbetrieb des Vergasers 2, d.h. nachdem in den einzelnen Zonen 64, 66 und vor allem 68 der Temperaturbereich des unterbrechungsfreien Normalbetriebs erreicht ist, wird die durchgesetzte Menge bei der vorliegenden Ausgestaltung durch das Gassaugvermögen der Gaspumpe 44 bestimmt, die mit einer vorgegebenen Förderrate das Verschwelungsgas aus der Brennkammer 68 über den Gasreiniger 4 abzieht. Über die oben beschriebene Regelung wird der Brennkammer 68 Material nachgeführt, so dass ständig ausreichend Material für die Verschwelung zur Verfügung steht. Der Luftsauerstoff wird durch die Leitung 80 ebenfalls durch das Saugvermögen der Pumpe 44 angesaugt, so dass der Oxidations- und Reduktionsprozess aufeinander abgestimmt sind. Die Sauerstoffzufuhr kann beispielsweise noch durch Anpassung des Strömungswiderstands mittels des Stellventil 82 angepasst werden - falls die Sauerstoffzufuhr nicht in Ausgestaltung über einen Abgaswert (z.B. Lambda-Wert) geregelt wird (s.o.).

Wie oben beschrieben, wird in die Trocknungskammer 64 das Abgas des Blockheizkraftwerks 50 eingeleitet. Anstelle des BHKWs kann auch eine andere Verbrennungsmaschine oder ein anderer abgaserzeugender Verbraucher als 'Abgaslieferant' angeschlossen sein. Das zugeführte Abgas dient der Erwärmung des in die Kammer 64 zugeführten Materials. Aufgrund des leichten Überdrucks des Abgases wird die Kammer 64 nach Außen gegen Luftsauerstoff abgeschirmt und eine Rückströmung von Verschwelungsgasen seitens der Pyrolysekammer 66 wird unterdrückt.

Der in der Trocknungskammer 64 entstehende Wasserdampf und das zugeführte Abgas werden gedrosselt durch eine Auslassleitung 100 ausgelassen, wobei der Wasserdampf durch das unter leichtem Überdruck durch die Leitung 52 zugeführte Abgas mitgeführt wird. Optional kann der Austrag des Wasserdampfes und des Abgases durch eine Förderpumpe 98 geregelt oder gesteuert werden. Die Pumpe 98 kann dabei durch Einstellen der Fördermenge die Menge des ausgelassenen Abgases und Wasserdampfes drosseln oder erhöhen. Anstelle oder zusätzlich zur Pumpe 98 ist ein Dosierventil 96 in einer Leitung 94 vorgesehen. Die Dampfleitung 94 verbindet die Kammer 64 mit der Brennkammer 68 und durch das Einstellen der Strömungsmenge mittels des Ventils 96 kann der Brennkammer 68 dosiert Wasserdampf (und Abgas) zugeführt werden. Dabei kann das Ventil 96 unter der Steuerung der Steuereinheit 110 im Auf-/Zu-Modus arbeiten, bei dem das Ventil bei Bedarf geöffnet und ansonsten geschlossen wird. Oder das Ventil 96 kann so angesteuert werden, dass eine von der Steuereinheit 110 vorgebbare Menge Wasserdampf zugeführt wird, oder so geregelt werden, dass die Dampfinenge eine Stellgröße zur Regelung einer Regelgröße ist. Im vorliegenden Fall ist die Regelgröße die Temperatur in der Brennzone 74, die in einem vorgegebenen Bereich - beispielsweise von 1.200 bis 1.400°C - gehalten werden soll. Wird von der Steuereinrichtung 110 über einen Temperatursensor (nicht dargestellt) erfasst, dass die Temperatur über den vorgegebenen Temperaturbereich steigt, wird das Ventil 96 geöffnet (und ggf. die Pumpe 98 eingeschaltet, falls noch nicht erfolgt oder nicht vorhanden), so dass aus der Trocknungskammer 64 Wasserdampf zur Abkühlung des Verbrennungsvorgangs in der Brennzone 74 zugeführt wird. Neben der Abkühlung wird das zugeführte Wasser auch zur H₂- und CH₄-Erzeugung beim Reduktionsprozess benötigt.

Die aus dem Gasreiniger 4 entnommene teerhaltige Lösungsphase c (siehe oben) wird von der Pumpe 40 aus dem Trennbehälter 8 abgesaugt und über die Leitung 28 in die Brennkammer 68 eingespritzt. Die teerhaltige Lösungsphase enthält die vom organischen Lösungsmittel zersetzten Endprodukte der ursprünglich als Teer (Gasphase und/oder Kondensat und/oder Ascheanlagerungen) in den Gaswäscher 6 zugeführten Verschwelungsprodukte. In der teerhaltigen Lösungsphase c liegen die C-Verbindungen nur noch als kurze Ketten oder vollständig zu Einzel-C-Verbindungen aufgelöst vor, so dass normalerweise kein Cracken der Teerverbindungen in der Brennzone 74 erforderlich ist. Dennoch können durch die Rückführung Teerpartikel in die Brennzone 74 gelangen, wo diese jedoch dann thermisch gecrackt werden und ebenfalls wie die anderen Bestandteile der teerhaltigen Lösungsphase bevorzugt in CO, CH₄ und CO₂ umgewandelt werden.

Alternativ kann die teerhaltige Lösungsphase c statt in die Brennkammer 68 in die Pyrolysekammer 68 zugeführt werden. Dort können die gelösten Teerverbindungen (die in der Regel kein oder sehr wenig Teer enthalten) zunächst dem Verschwelungsprozess und nach Überführung in die Kammer 68 durch die Schleuse 92 dem Oxidations- und Reduktionsprozess unterzogen werden.

Die beim Verbrennungsprozess entstehende Asche wird zumindest teilweise mit den Verschwelungsgasen über die Leitung 20 aus dem Brennraum 68 abgezogen und wie oben beschrieben im Wäscher aus dem Verschwelungsgasstrom entfernt. In Abhängigkeit von dem dem Pyrolysator zugeführten Ausgangsmaterial kann der Ascheanteil nach der Verbrennung in der Kammer 68 so gering sein, dass die Asche vollständig vom Verschwelungsgasstrom mitgeführt und so aus der Kammer 68 entfernt wird. Dann müssen aus der Kammer 68 keine festen Rückstände separat entfernt werden. Entsteht jedoch bei der Verbrennung ein hoher Ascheanteil (beispielsweise bei der Verwertung von Papierschliff im Pyrolysator, beim dem ein sehr hoher Calciumanteil vorliegt), dann muss die sich am Boden ablagernde Asche von Zeit zu Zeit aus der Brennkammer 68 über einen nicht dargestellten Ascheaustrag entfernt werden. Hierfür ist eine Schleuse oder ein Austragmechanismus (z.B. Schnecke) vorgesehen, die bzw. der verhindert, dass beim Abführen der Asche Umgebungsluft unkontrolliert in die Kammer 68 gelangt.

Dadurch, dass die Zonen bzw. Kammern (62) 64, 66 und 68 durch die Trennwände (63) 65 und 67 voneinander getrennt sind, sind die Lage der Zonen und die Prozessbedingungen in den Zonen weitgehend festgelegt. Durch die Betätigung der Verschlüsse bzw. Schleusen (86) 90 und 92 ist die Materialzufuhr bzw. -fluss von der einen Zone in die nächste Zone steuerbar, was die Kontrolle der Prozessbedingungen ermöglicht. Vor allem der Pyrolyseprozess in der Pyrolysekammer ist unabhängig vom Oxidations- und Reduktionsprozess in der Brennkammer einstellbar und umgekehrt, so dass der gesamte als Verschwelungsprozess bezeichnete Vorgang im Vergaser 2 bei einer Vielfalt von unterschiedlichen Ausgangsmaterialien oder bei wechselnden Ausgangsmaterialien einsetzbar und optimierbar ist.

Die Pyrolyse in Kammer 66 liefert der Brennkammer das verbrennbare Material in einer aufbereiteten Form, die es erlaubt den Verbrennungsprozess optimal und ohne Verwendung eines Festbetts in einer Wirbelzonenoxidation und -reduktion durchzuführen.

Gemäß einem weiteren Erfindungsaspekt ist ein Vergaser zur Vergasung von kohlenstoffhaltigem Material mit den folgenden Merkmalen und Ausgestaltungen vorgesehen. Dabei ist dieser Vergaser oben beschrieben und kann Teile oder Kombinationen der obigen Ausgestaltungen aufweisen:
Gemäß dem weiteren Aspekt der Erfindung weist der Vergaser (2) zur Vergasung von kohlenstoffhaltigem Material, insbesondere von organischen und/oder anorganischen Abfällen, auf:
   eine Trocknungskammer (64) mit einer Beschickungsöffnung (86) zum Trocknen des durch die Beschickungsöffnung zugeführten kohlenstoffhaltigen Materials, und
   eine Pyrolysekammer (66) zur teilweise thermischen Umsetzung und Vergasung des in der Trocknungskammer (64) getrockneten Materials, wobei das getrocknete Material von der Trocknungskammer durch zumindest eine Öffnung (90), vorzugsweise durch zumindest eine verschließbare Öffnung oder Schleuse, in die Pyrolysekammer überführbar ist. Der Vergaser ist gekennzeichnet durch: eine Brennkammer (68) zur Umsetzung des thermisch behandelten Materials aus der Pyrolysekammer (66) unter Sauerstoffzugabe, wobei das thermisch behandelte Material von der Pyrolysekammer (66) durch zumindest eine Öffnung (92), vorzugsweise durch zumindest eine verschließbare Öffnung oder Schleuse, in die Brennkammer überführbar ist, und wobei die Brennkammer (68) eine Brennzone (74), insbesondere eine Wirbelschichtbrennzone aufweist.
Bei einer Ausführungsform des weiteren Aspekts ist an die Trocknungskammer (64) eine Abgasleitung (52) zur Zuführung von Abgas angeschlossen, wobei mittels des zugeführten Abgases die Trocknungskammer gegen Umgebungssauerstoff abgedichtet wird und/oder das zugeführte kohlenstoffhaltige Material getrocknet wird. Bei einer Ausführungsform davon ist das durch die Abgasleitung (52) zugeführte Abgas das Abgas eines Verbrennungsmotors (50) und/oder eines Heizbrenners, wobei insbesondere der Verbrennungsmotor und/oder der Heizbrenner mittels des Verschwelungsgases des Vergasers oder eines Folgeprodukts des Verschwelungsgases des Vergasers betreibbar ist. Bei einer Ausführungsform davon verbindet eine Fluidleitung (94) die Trocknungskammer (64) mit der Brennkammer (68), so dass Wasserdampf und/oder Prozessgase aus der Trocknungskammer in die Brennkammer überführbar sind. Bei einer Ausführungsform davon ist der Fluidleitung (94) eine Dosiereinrichtung (96) und/oder eine Pumpeinrichtung (98) zugeordnet, so dass der Fluidstrom von der Trocknungskammer (64) zur Brennkammer (68) durch Ansteuerung der Dosiereinrichtung und/oder der Pumpeinrichtung mittels einer Steuereinrichtung (110) steuerbar oder regelbar ist. Bei einer Ausführungsform davon ist mittels der Steuereinrichtung (110) der Fluidstrom in Abhängigkeit der Temperatur und/oder des Lambdawertes der Verbrennung in der Brennkammer (68) einstellbar oder regelbar. Bei einer Ausuhrungsform davon, ist die Beschickungsöffnung (86) zumindest eine verschließbare Öffnung oder als zumindest eine Schleuse ausgebildet, durch die das kohlenstoffhaltige Material von außerhalb des Vergasers (2) der Trocknungskammer (64) zuführbar ist. Bei einer Ausführungsform davon ist der Brennkammer (68) durch eine Sauerstoffleitung oder -öffnung (80) Luft oder Sauerstoff zuführbar und vorzugsweise ist die Sauerstoffleitung durch die Trocknungskammer (64) und/oder durch die Pyrolysekammer (66) geführt. Bei einer Ausführungsform davon ist zumindest ein Betätigungselement (80, 84; 88) vorgesehen, das zur Betätigung
eines Verschließelements der Öffnung oder Schleuse (86) zwischen der Außenseite des Vergasers (2) und der Trocknungskammer (64) wirkt,
eines Verschließelements der Öffnung oder Schleuse (90) zwischen der Trocknungskammer (64) und der Pyrolysekammer (66) wirkt, und/oder
eines Verschließelements der Öffnung oder Schleuse (92) zwischen der Pyrolysekammer (66) und der Brennkammer (68) wirkt. Bei einer Ausführungsform davon ist das Betätigungselement (80, 84) zeitweise von dem oder einem der Verschließelemente der Öffnungen oder der Schleusen (90, 92) abkoppelbar, so dass bei Betätigung einer oder mehrerer der anderen Verschließelemente das abgekoppelte Verschließelement nicht betätigt wird, wobei insbesondere das abkoppelbare Verschließelement der Öffnung oder Schleuse zwischen der Pyrolysekammer (66) und der Brennkammer (68) zugeordnet ist.

Gemäß einem noch weiteren Aspekt der Erfindung ist eine Vergaseranordnung (2, 4) vorgesehen mit einem Vergaser (2) und einer dem Vergaser nachgeschaltete Gasreinigungseinrichtung (4) die Verschwelungsgase aus dem Vergaser, wobei mittels der Gasreinigungseinrichtung (4) Teerbestandteile aus dem Verschwelungsgas entfernbar sind, wobei zwischen der Gasreinigungseinrichtung (4) und dem Vergaser (2) eine Rückführleitung (28) angeordnet ist, und wobei die Rückführleitung (28) die abgesonderten Teerbestandteile und/oder die in der Gasreinigungseinrichtung (4) umgesetzten Teerbestandteile in den Vergaser (2) zurückführt. Bei einer Ausführungsform davon führt die Rückführleitung (28) die abgesonderten Teerbestandteile und/oder die umgesetzten Teerbestandteile in die Pyrolysekammer (66) oder die Brennkammer (68) zurück oder sprüht diese ein. Bei einer Ausführungsordnung davon weist die Gasreinigungseinrichtung (4) einen Gaswäscher (6) auf, der vorzugsweise zumindest teilweise mit einem organischen Lösungsmittel gefüllt ist, insbesondere mit einem organischen Öl, mit Methylester, Glycerin und/oder mit Rapsmethylester (RME).

**Bezugszeichenliste**

| | | | |
|---|---|---|---|
| 2 | Pyrolysator | 64 | Trocknungskammer |
| 4 | Gasreiniger | 65 | zweite Trennwand |
| 6 | Gaswäscher | 66 | Pyrolysekammer |
| 8 | Trennbehälter | 67 | dritte Trennwand |
| 10 | Fermenter | 68 | Brennkammer |
| 20 | Verschwelungsgasleitung | 70 | Gebläsemotor |
| 21 | Materialzufuhr | 72 | Gebläse |
| 22 | Gasleitung | 74 | Brennzone |
| 24 | Ablaufleitung | 80 | Sauerstoffleitung |
| 26 | Lösungsmittelleitung | 82 | Schieber |
| 28 | Teerlösungsleitung | 84 | Manipulator |
| 30 | Absetzleitung | 86 | erste Schleuse |
| 31 | Biomassezufuhr | 88 | Manipulator |
| 32 | Biogasleitung | 90 | zweite Schleuse |
| 33 | Düngeraustragleitung | 92 | dritte Schleuse |
| 34 | opt. Feststofffilter | 94 | Dampfleitung |
| 36 | opt. Leitung wässrige Lösung | 96 | Dosierventil |
| 38 | Feststoffleitung | 98 | Förderpumpe |
| 40 | Teerlösungspumpe | 100 | Auslass |
| 42 | Umwälzpumpe | 110 | Steuereinheit |
| 44 | Gaspumpe | | |
| 46 | Pumpe für wässrige Lösung | a | Waschlösung |
| 50 | Blockheizkraftwerk | b | belastete Waschlösung |
| 52 | Abgasleitung | c | teerhaltige Lösungsphase |
| 54 | Verbraucherleitung | d | organisches Lösungsmittel |
| 62 | Zuführung | e | wässrige Lösungsphase |
| 63 | erste Trennwand | f | Feststoff-angereichterte Phase |

## Patentansprüche

1. Kombi-Mischgasanlage zur Erzeugung von Wasserstoff- und/oder methanhaltigem Gas, die aufweist:
eine Verschwelungseinrichtung (2), insbesondere einen Vergasungsreaktor, zur Erzeugung von Verschwelungsgasen, und
eine der Verschwelungseinrichtung (2) nachgeschaltete Gasreinigungseinrichtung (4) zur Reinigung der Verschwelungsgase,
wobei die Gasreinigungseinrichtung (4) dazu ausgelegt ist, Teerbestandteile (c) aus den Verschwelungsgasen zu entfernen, und
wobei zwischen der Gasreinigungseinrichtung (4) und der Verschwelungseinrichtung (2) eine Rückführleitung (28) verläuft, mit der die Teerbestandteile (c) von der Gasreinigungseinrichtung (4) in die Verschwelungseinrichtung (2) zurückleitbar sind,
**dadurch gekennzeichnet, dass**
die Reinigungseinrichtung (4) einen Gaswäscher (6) mit einem Reinigungsmedium (a), das nicht-gasförmige Bestandteile aus dem Verschwelungsgasstrom aussondert, und eine separat vom Gaswäscher (6) angeordnete Trenneinheit (8) aufweist, in der sich die aus dem Verschwelungsgasstrom ausgesonderten, nicht-gasförmigen Bestandteile in verschiedene Phasen trennen.

2. Kombi-Mischgasanlage nach Anspruch 1, wobei sich in der Trenneinheit (8)
- eine teerhaltige Lösungsmittelphase (c) vom Reinigungsmedium (a) des Gaswäschers (6) trennt, und/oder
- die ausgefällten Feststoffbestandteile (f) vom Reinigungsmedium absetzen oder ausfällen, und/oder
- kondensiertes Wasser vom Reinigungsmedium trennt, und/oder
- eine wässrige Lösung (e) von wasserlöslichen nicht-gasförmigen Bestandteilen vom Reinigungsmedium trennt.

3. Kombi-Mischgasanlage nach Anspruch 1 oder 2, wobei das Reinigungsmedium (a) Teerbestandteile (c) aus den Verschwelungsgasen löst, wobei das Reinigungsmedium vorzugsweise ein organisches Lösungsmittel ist.

4. Kombi-Mischgasanlage nach Anspruch 1, 2 oder 3, wobei die Gasreinigungseinrichtung (4) weiterhin dazu ausgelegt ist, mittels des Reinigungsmediums (a) Feststoffbestandteile (f) und/oder wasserlösliche Stoffe (e) und/oder Wasserdampf aus den Verschwelungsgasen zu entfernen.

5. Kombi-Mischgasanlage nach einem der vorhergehenden Ansprüche, wobei der Gaswäscher (6) und die Trenneinheit (8) so fluid miteinander kommunizieren, insbesondere über Verbindungsleitungen (24, 26) so miteinander verbunden sind, dass ein Medium-Fluidkreislauf zwischen Gaswäscher und Trenneinheit hergestellt ist, wobei insbesondere das durch die Gaswäsche verunreinigte Reinigungsmedium (a) dem Gaswäscher (6) entnommen und der Trenneinheit (8) zugeführt wird und gereinigtes Reinigungsmedium (d) der Trenneinheit (8) entnommen und dem Gaswäscher zugeführt wird.

6. Kombi-Mischgasanlage nach Anspruch 5, wobei die Gasreinigungseinrichtung (4) eine erste Verbindungsleitung (24) zwischen dem Gaswäscher (6) und der Trenneinheit (8) zum Überführen des durch die Gaswäsche verunreinigten Reinigungsmediums in eine erste Trennzone der Trenneinheit, und eine zweite Verbindungsleitung (26) zwischen der Trenneinheit und dem Gaswäscher zur Rückführung des durch Phasentrennung in einer zweiten Trennzone der Trenneinheit gereinigten bzw. angereicherten Reinigungsmediums in den Gaswäscher aufweist.

7. Kombi-Mischgasanlage nach einem der vorhergehenden Ansprüche, wobei die Gasreinigungseinrichtung (4), insbesondere der Gaswäscher (6), zumindest teilweise mit einem vorzugsweise organischen Lösungsmittel (d) als Reinigungsmedium gefüllt ist, insbesondere mit einem organischen Öl, mit Methylester oder mit Rapsmethylester (RME).

8. Kombi-Mischgasanlage nach einem der vorhergehenden Ansprüche, wobei der Gasreinigungseinrichtung (4) eine Fermentationseinrichtung (10) nachgeschaltet ist zur Umsetzung der gereinigten Verschwelungsgase in angereichertes Wasserstoff- und/oder methanhaltiges Gas.

9. Kombi-Mischgasanlage nach Anspruch 8, wobei zwischen der Reinigungseinrichtung (4), insbesondere zwischen der Trenneinheit (8), und der Fermentationseinrichtung (10) eine Leitung (30) zum Zuführen einer wässrigen Lösung mit darin gelösten, wasserlöslichen Stoffen (e) und/oder Feststoffbestandteilen (f) aus der Reinigungseinrichtung (8) in die Fermentationseinrichtung (10) verläuft.

10. Kombi-Mischgasanlage nach Anspruch 8 oder 9, wobei die Fermentationseinrichtung (10) dazu ausgelegt ist, Biomasse aufzunehmen, um daraus zusammen mit dem gereinigten Verschwelungsgas und/oder den Feststoffbestandteilen (f) und/oder der wässrigen Lösung mit darin gelösten, wasserlöslichen Stoffen (e) das Wasserstoff- und/oder methanhaltige Gas zu erzeugen.

11. Verfahren zur Erzeugung von Wasserstoff- und/oder methanhaltigem Gas in einer Kombi-Mischgasanlage nach einem der vorherigen Ansprüche, wobei die Kombi-Mischgasanlage aufweist:
eine Verschwelungseinrichtung (2), insbesondere einen Vergasungsreaktor, und
eine der Verschwelungseinrichtung (2) nachgeschaltete Gasreinigungseinrichtung (4),
wobei das Verfahren die Schritte aufweist:
thermische Behandlung von kohlenstoffhaltigem Material in der Verschwelungseinrichtung (2) zur Erzeugung von Verschwelungsgasen,
Einleitung der Verschwelungsgase von der Verschwelungseinrichtung (2) in die Reinigungseinrichtung (4), und
Reinigung der Verschwelungsgase in der Reinigungseinrichtung (4), wobei die Reinigungseinrichtung zumindest die im Verschwelungsgasstrom von der Verschwelungseinrichtung mitgeführten Teerbestandteile mittels des Reinigungsmediums aus dem Verschwelungsgasstrom entfernt,
**dadurch gekennzeichnet,**
**dass** die Gasreinigungseinrichtung einen Gaswäscher (6) und eine separate Trenneinheit (8) aufweist, in der sich die aus dem Verschwelungsgasstrom ausgesonderten nicht-gasformigen Bestandteile in verschiedenen Phasen trennen und dass in der Gasreinigungseinrichtung (4) ein vorzugsweise organisches Lösungsmittel (d) als Reinigungsmedium zum Überführen der Teerbestandteile in eine teerhaltige Lösungsmittelphase (c) eingesetzt ist, und
**gekennzeichnet durch** den Schritt
Zuführen der teerhaltigen Lösungsmittelphase (c) zur Verschwelungseinrichtung (2).

12. Verfahren nach Anspruch 11, wobei in der Trenneinheit (8) das Reinigungsmedium, das im Gaswäscher (6) benötigt wird, oder ein Bestandteil des Reinigungsmediums, durch eine Phasentrennung angereichert und zur Reinigung des Verschwelungsgasstroms in den Gaswäscher zurückgeführt wird.

13. Verfahren nach Anspruch 11 oder 12, wobei das Reinigen der Verschwelungsgase weiterhin
das Kondensieren des im Verschwelungsgasstrom mitgeführten Wasserdampfes,
das Überführen von im Verschwelungsgasstrom mitgeführten wasserlöslichen Bestandteilen in eine wässrige Lösung (e), und/oder
das Abscheiden von im Verschwelungsgasstrom mitgeführten Feststoffen (f) umfasst.

14. Verfahren nach einem der Ansprüche 11 bis 13, wobei der Gasreinigungseinrichtung (4) eine Fermentierungseinrichtung (10) nachgeschaltet ist und das Verfahren weiterhin aufweist: Zuführen der gereinigten Verschwelungsgase in die Fermentierungseinrichtung (10) zur Umsetzung der gereinigten Verschwelungsgase in angereichertes Wasserstoff- und/oder methanhaltiges Gas.

15. Verfahren nach Anspruch 14 in Rückbezug auf Anspruch 13, wobei die wässrige Lösung (e) mit den wasserlöslichen Bestandteilen der Fermentierungseinrichtung (10) zugeführt wird, und/oder die abgeschiedenen Feststoffe (f) ausgefiltert und/oder der Fermentierungseinrichtung (10) zugeführt werden.

## Claims

1. Combined mixed gas system for generating hydrogen and/or methane containing gas, which comprises:
a carbonizing device (2), in particular a gasification reactor, for generating carbonization gases, and
a gas cleaning device (4) downstream of the carbonizing device (2) for purifying the carbonization gases,
wherein the gas cleaning device (4) is adapted to remove tar constituents (c) from the carbonization gases, and
wherein a return line (28) extends between the gas cleaning device (4) and the carbonizing device (2), by which the tar constituents (c) can be returned from the gas cleaning device (4) into the carbonizing device (2),
**characterized in that**
the cleaning device (4) comprises a gas scrubber (6) with a cleaning medium (a) separating non-gaseous constituents from the carbonization gas stream, and a separating unit (8) disposed separately from the gas scrubber (6), in which the non-gaseous constituents separated from the carbonization gas stream split into different phases.

2. Combined mixed gas system according to claim 1, wherein in the separating unit (8)
- a tar containing solvent phase (c) separates from the cleaning medium (a) of the gas scrubber (6), and/or
- the precipitated solid constituents (f) sediment or precipitate from the cleaning medium, and/or
- condensed water separates from the cleaning medium, and/or
- an aqueous solution (e) of water-soluble non-gaseous constituents separates from the cleaning medium.

3. Combined mixed gas system according to claim 1 or 2, wherein the cleaning medium (a) dissolves tar constituents (c) from the carbonization gases, wherein the cleaning medium is preferably an organic solvent.

4. Combined mixed gas system according to claim 1, 2 or 3, wherein the gas cleaning device (4) is further adapted to remove solid constituents (f) and/or water-soluble substances (e) and/or water vapor from the carbonization gases by means of the cleaning medium (a).

5. Combined mixed gas system according to any one of the preceding claims, wherein the gas scrubber (6) and the separating unit (8) fluidly communicate with each other, in particular are connected to each other via connecting lines (24, 26), such that a medium fluid circuit is established between gas scrubber and separating unit, wherein in particular the cleaning medium (a) contaminated by the gas scrubbing is removed from the gas scrubber (6) and supplied to the separating unit (8) and purified cleaning medium (d) is removed from the separating unit (8) and supplied to the gas scrubber.

6. Combined mixed gas system according to claim 5, wherein the gas cleaning device (4) comprises a first connecting line (24) between the gas scrubber (6) and the separating unit (8) for transferring the cleaning medium contaminated by the gas scrubbing into a first separating zone of the separating unit, and a second connecting line (26) between the separating unit and the gas scrubber for returning the cleaning medium purified and enriched, respectively, by phase separation in a second separating zone of the separating unit into the gas scrubber.

7. Combined mixed gas system according to any one of the preceding claims, wherein the gas cleaning device (4), in particular the gas scrubber (6), is at least partially filled with a preferably organic solvent (d) as the cleaning medium, in particular with an organic oil, with methyl ester or rapeseed methyl ester (RME).

8. Combined mixed gas system according to any one of the preceding claims, wherein a fermentation device (10) is disposed downstream of the gas cleaning device (4) for converting the purified carbonization gases into enriched hydrogen and/or methane containing gas.

9. Combined mixed gas system according to claim 8, wherein a line (30) for supplying an aqueous solution with water-soluble substances (e) dissolved therein and/or solid constituents (f) from the cleaning device (8) into the fermentation device (10) extends between the cleaning device (4), in particular between the separating unit (8), and the fermentation device (10).

10. Combined mixed gas system according to claim 8 or 9, wherein the fermentation device (10) is adapted to receive biomass to generate the hydrogen and/or methane containing gas therefrom together with the purified carbonization gas and/or the solid constituents (f) and/or the aqueous solution with water-soluble substances (e) dissolved therein.

11. Method for generating hydrogen and/or methane containing gas in a combined mixed gas system according to any one of the preceding claims, wherein the combined mixed gas system comprises:
a carbonizing device (2), in particular a gasification reactor, and
a gas cleaning device (4) downstream of the carbonizing device (2),
wherein the method comprises the steps of:
thermally treating carbon containing material in the carbonizing device (2) for generating carbonization gases,
introducing the carbonization gases from the carbonizing device (2) into the cleaning device (4), and
purifying the carbonization gases in the cleaning device (4), wherein the cleaning device at least removes the tar constituents carried along in the carbonization gas stream from the carbonizing device by means of the cleaning medium from the carbonization gas stream,
**characterized in that**
the gas cleaning device comprises a gas scrubber (6) and a separate separating unit (8), in which the non-gaseous constituents separated from the carbonization gas stream split into different phases, and
a preferably organic solvent (d) is employed as the cleaning medium for transferring the tar constituents into a tar containing solvent phase (c) in the gas cleaning device (4), and
**characterized by** the step of
supplying the tar containing solvent phase (c) to the carbonizing device (2).

12. Method according to claim 11, wherein the cleaning medium, which is required in the gas scrubber (6), or a constituent of the cleaning medium, is enriched by phase separation in the separating unit (8) and returned into the gas scrubber for cleaning of the carbonization gas stream.

13. Method according to claim 11 or 12, wherein the cleaning of the carbonization gases further includes
condensing the water vapor carried along in the carbonization gas stream,
transferring water-soluble constituents carried along in the carbonization gas stream into an aqueous solution (e), and/or
separating solids (f) carried along in the carbonization gas stream.

14. Method according to any one of claims 11 to 13, wherein a fermentation device (10) is disposed downstream of the gas cleaning device (4) and the method further comprises: supplying the purified carbonization gases into the fermentation device (10) for converting the purified carbonization gases into enriched hydrogen and/or methane containing gas.

15. Method according to claim 14 in dependency of claim 13, wherein the aqueous solution (e) with the water-soluble constituents is supplied to the fermentation device (10) and/or the separated solids (f) are filtered out and/or supplied to the fermentation device (10).

## Revendications

1. Installation combinée à gaz mixte pour la production de gaz contenant de l'hydrogène et/ou du méthane, qui présente :
un dispositif de pyrolyse (2), en particulier un réacteur de gazéification, pour produire des gaz de pyrolyse, et
un dispositif de purification de gaz (4) en aval du dispositif de pyrolyse (2) pour purifier les gaz de pyrolyse,
dans laquelle le dispositif de purification de gaz (4) est conçu pour éliminer les composants de goudron (c) des gaz de pyrolyse, et
dans laquelle une conduite de retour (28) s'étend entre le dispositif de purification de gaz (4) et le dispositif de pyrolyse (2), par laquelle les composants de goudron (c) peuvent être renvoyés du dispositif de purification de gaz (4) dans le dispositif de pyrolyse (2),
**caractérisée en ce que**
le dispositif de purification (4) présente un laveur de gaz (6), avec un milieu de purification (a) qui élimine les composants non gazeux du flux de gaz de pyrolyse, et une unité de séparation (8) disposée séparément du laveur de gaz (6), dans laquelle les composants non gazeux éliminés du flux de gaz de pyrolyse se séparent en différentes phases.

2. Installation combinée à gaz mixte selon la revendication 1, dans laquelle, dans l'unité de séparation (8) :
- une phase de solvant contenant du goudron (c) se sépare du milieu de purification (a) du laveur de gaz, (6) et/ou
- les composants solides (f) précipités se déposent ou précipitent, et/ou
- l'eau condensée se sépare du milieu de purification, et/ou
- une solution aqueuse (e) de composants non gazeux hydrosolubles se sépare du milieu de purification.

3. Installation combinée à gaz mixte selon la revendication 1 ou 2, dans laquelle le milieu de purification (a) dissout les composants de goudron (c) des gaz de pyrolyse, le milieu de purification étant de préférence un solvant organique.

4. Installation combinée à gaz mixte selon la revendication 1, 2 ou 3, dans laquelle le dispositif de purification de gaz (4) est en outre conçu pour éliminer au moyen du milieu de purification (a) les composants solides (f) et/ou les substances hydrosolubles (e) et/ou la vapeur d'eau des gaz de pyrolyse.

5. Installation combinée à gaz mixte selon l'une des revendications précédentes, dans laquelle le laveur de gaz (6) et l'unité de séparation (8) communiquent entre eux fluidiquement, en particulier sont reliés entre eux par des conduites de raccordement (24, 26), de telle sorte qu'un circuit de fluide de milieu est établi entre le laveur de gaz et l'unité de séparation, en particulier le milieu de purification (a) contaminé par le lavage de gaz étant prélevé du laveur de gaz (6) et amené à l'unité de séparation (8) et du milieu de purification purifié (d) prélevé de l'unité de séparation (8) et amené au laveur de gaz.

6. Installation combinée à gaz mixte selon l'une des revendications précédentes, dans laquelle le dispositif de purification de gaz (4) présente une première conduite de raccordement (24) entre le laveur de gaz (6) et l'unité de séparation (8) pour transférer le milieu de purification contaminé par le lavage de gaz dans une première zone de séparation de l'unité de séparation et une deuxième conduite de raccordement (26) entre l'unité de séparation et le laveur de gaz pour ramener dans le laveur de gaz le milieu de purification purifié ou enrichi par séparation de phases dans une deuxième zone de séparation de l'unité de séparation.

7. Installation combinée à gaz mixte selon l'une des revendications précédentes, dans laquelle le dispositif de purification de gaz (4), en particulier le laveur de gaz (6), est rempli au moins en partie d'un solvant (d) de préférence organique en tant que milieu de purification, en particulier d'une huile organique, d'ester méthylique ou d'ester méthylique de colza (EMC).

8. Installation combinée à gaz mixte selon l'une des revendications précédentes ; dans laquelle le dispositif de purification de gaz (4) est suivi d'un dispositif de fermentation (10) pour transformer les gaz de pyrolyse purifiés en gaz enrichi contenant de l'hydrogène et/ou du méthane.

9. Installation combinée à gaz mixte selon la revendication 8, dans laquelle une conduite (30) s'étend entre le dispositif de purification de gaz (4), en particulier entre l'unité de séparation (8), et le dispositif de fermentation (10) pour amener une solution aqueuse contenant des substances hydrosolubles (e) dissoutes et/ou des composants solides (f) provenant du dispositif de purification (8) dans le dispositif de fermentation (10).

10. Installation combinée à gaz mixte selon la revendication 8 ou 9, dans laquelle le dispositif de fermentation (10) est conçu pour recevoir de la biomasse pour produire à partir de celle-ci, en combinaison avec le gaz de pyrolyse purifié et/ou les composants solides (f) et/ou la solution aqueuse contenant des substances hydrosolubles (e) dissoutes, le gaz contenant de l'hydrogène et/ou du méthane.

11. Procédé de production de gaz contenant de l'hydrogène et/ou du méthane dans une installation combinée à gaz mixte selon l'une des revendications précédentes, laquelle installation combinée à gaz mixte présente :
un dispositif de pyrolyse (2), en particulier un réacteur de gazéification, et
un dispositif de purification de gaz (4) en aval du dispositif de pyrolyse (2),
lequel procédé présente les étapes suivantes :
traitement thermique d'une matière carbonée dans le dispositif de pyrolyse (2) pour produire des gaz de pyrolyse,
introduction des gaz de pyrolyse du dispositif de pyrolyse (2) dans le dispositif de purification (4), et
purification des gaz de pyrolyse dans le dispositif de purification (4), le dispositif de purification éliminant au moins les composants de goudron (c) entraînés dans le flux de gaz de pyrolyse provenant du dispositif de pyrolyse du flux de gaz de pyrolyse au moyen du milieu de purification,
**caractérisé en ce**
**que** le dispositif de purification de gaz présente un laveur de gaz (6) et une unité de séparation (8) séparée, dans laquelle les composants non gazeux éliminés du flux de gaz de pyrolyse se séparent en différentes phases et
**qu'**un solvant (d) de préférence organique est utilisé dans le dispositif de purification de gaz (4) comme milieu de purification pour transformer les composants de goudron en une phase de solvant contenant du goudron (c), et
**caractérisé par** l'étape suivante :
amenée de la phase de solvant contenant du goudron (c) au dispositif de pyrolyse (2).

12. Procédé selon la revendication 11, dans lequel, dans l'unité de séparation (8), le milieu de purification qui est nécessaire dans le laveur de gaz (6), ou un composant du milieu de purification, est enrichi par une séparation de phases et ramené dans le laveur de gaz pour purifier le flux de gaz de pyrolyse.

13. Procédé selon la revendication 11 ou 12, dans lequel la purification des gaz de pyrolyse comprend en outre :
la condensation de la vapeur d'eau entraînée dans le flux de gaz de pyrolyse,
la transformation de composants hydrosolubles entraînés dans le flux de gaz de pyrolyse en une solution aqueuse (e), et/ou
le dépôt des matières solides (f) entraînées dans le flux de gaz de pyrolyse.

14. Procédé selon l'une des revendications 11 à 13, dans lequel le dispositif de purification de gaz (4) est suivi d'un dispositif de fermentation (10) et le procédé comprend en outre l'étape suivante : amenée des gaz de pyrolyse purifiés dans le dispositif de fermentation (10) pour transformer les gaz de pyrolyse purifiés en gaz enrichi contenant de l'hydrogène et/ou du méthane.

15. Procédé selon la revendication 14 lorsqu'elle se réfère à la revendication 13, dans lequel la solution aqueuse (e) contenant les composants hydrosolubles est amenée au dispositif de fermentation (10) et/ou les matières solides (f) déposées sont éliminées par filtration et/ou amenées au dispositif de fermentation (10).
